(19) Europäisches Patentamt
European Patent Office
Office européen des brevets

(11) Publication number: 0 192 328 B1

# (12) EUROPEAN PATENT SPECIFICATION

(45) Date of publication of patent specification: 09.05.90

(51) Int. Cl.$^5$: C 07 D 491/048, B 41 M 5/124

(21) Application number: 86300305.9

(22) Date of filing: 17.01.86

## (54) Chromogenic compounds.

(30) Priority: 22.02.85 GB 8504631

(43) Date of publication of application: 27.08.86 Bulletin 86/35

(45) Publication of the grant of the patent: 09.05.90 Bulletin 90/19

(84) Designated Contracting States: CH DE FR GB IT LI

(56) References cited:
CH-A- 645 112
GB-A-1 367 569
GB-A-2 141 729
US-A-4 508 897

(73) Proprietor: IMPERIAL CHEMICAL INDUSTRIES PLC
Imperial Chemical House, Millbank
London SW1P 3JF (GB)

(72) Inventor: Hall, Nigel
23 Underwood Way
Shaw Oldham Lancashire (GB)

(74) Representative: Stephenson, Kenneth et al
Imperial Chemical Industries PLC Legal Department: Patents PO Box 6 Bessemer Road
Welwyn Garden City Herts, AL7 1HD (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 192 328 B1

## Description

This invention relates to chromogenic compounds and in particular to chromogenic pyrazine compounds. A chromogenic compound is a colourless or nearly colourless organic material which is capable of undergoing a colour-forming reaction on being brought into contact with a suitable co-reactant, for example an electron-accepting co-reactant such as an active clay material.

This colour-forming reaction may take place by contacting the chromogenic compound, preferably in the form of a solution in a suitable organic solvent, with the solid co-reactant, which may for example be coated onto or incorporated into a paper material. For many applications, a solution of the chromogenic compound in a suitable organic solvent is separated from the solid co-reactant by a membrane so that the colour develops only when the membrane is ruptured. For example, pressure-sensitive recording materials generally consist of the combination of (i) a base sheet having coated thereon microcapsules containing a solution in an organic solvent of the chromogenic compound and (ii) a receiving sheet coated with a suitable co-reactant. When pressure is applied to the base sheet, as for example by handwriting or typewriting, the membranes of the microcapsules at the point of pressure are ruptured, and a colour-forming reaction takes place as the solution of the chromogenic compound contacts the co-reactant. Variations of this general principle are possible, as for example a single recording sheet having a coating of micro-encapsulated chromogenic compound solution and a coating of co-reactant. Heat-sensitive recording materials function in a similar manner, the chromogenic compound and the co-reactant being separated by a medium which melts on the application of local heat as described for example in United States Patent No 3,539,375.

One problem which is frequently encountered in the practical application of chromogenic materials is the development of colour, even in the absence of a co-reactant, when the chromogenic material is in contact with the base paper (see for example GB 2 141 729A — page 4). Thus for example when a microencapsulated layer is applied to the base paper during manufacture it is inevitable that some of the microcapsules will rupture, releasing the solution of the chromogenic material. It is clearly important that no colour is formed at this stage.

Chromogenic pyrazine lactone (5,7-dihydrofuro [3,4,1-b]-pyrazin-5-one) compounds are disclosed in GB 1,367,569 and in CH—A—645 112 but these compounds have been found to suffer from the above-mentioned problem of the development of colour in contact with base papers.

According to the present invention, there is provided a chromogenic (5,7-dihydrofuro [3,4,1-b]-pyrazin-5-one compound having substituents in one or both of the 2,3- positions.

Thus, the present invention provides a chromogenic pyrazine compound of formula:—

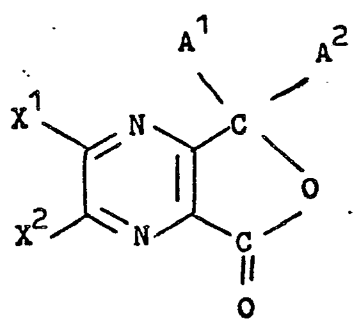

wherein $X^1$ and $X^2$, which may be the same or different, are each selected from hydrogen, alkyl, optionally substituted alkyl, alkoxy, carboxyalkyl, optionally substituted aryl, optionally substituted heteroaromatic, optionally substituted aralkyl or dialkylamino, provided that both $X^1$ and $X^2$ are not both hydrogen at the same time; and $A^1$ and $A^2$, which may be the same or different, are each selected from groups of the formulae:

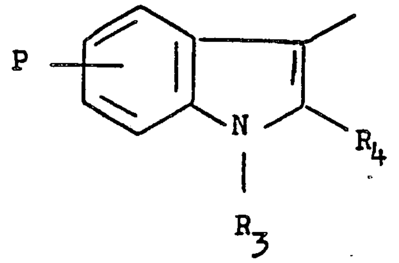

(II)

wherein $R_3$ and $R_4$ are each independently hydrogen, optionally substituted alkyl, optionally substituted aryl or optionally substituted aralkyl and P is hydrogen, alkyl, alkoxy , or halogen;

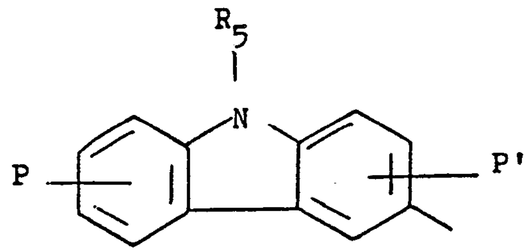

(III)

wherein P and P', which may be the same or different, are optional substituents having the meaning given above for P; and $R_5$ is hydrogen, alkyl, aryl or aralkyl;

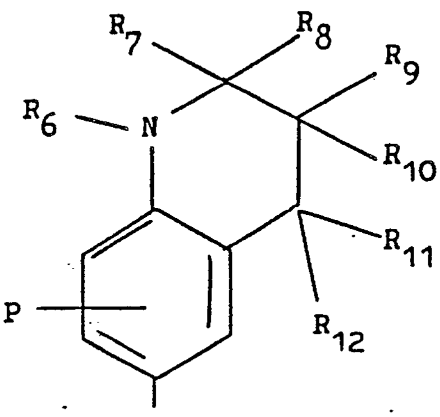

(IV)

wherein P is an optional substituent having the meaning given above, $R_6$ is hydrogen, alkyl, aryl or aralkyl, and the groups $R_7$ to $R_{12}$ are hydrogen, alkyl, aryl or aralkyl;

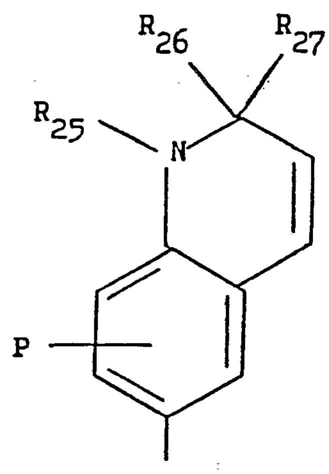

(IVa)

wherein P is an optional substituent having the meaning given above, $R_{25}$ is hydrogen or more preferably alkyl, aryl or aralkyl and the groups $R_{26}$ and $R_{27}$ are hydrogen, alkyl, aryl or aralkyl;

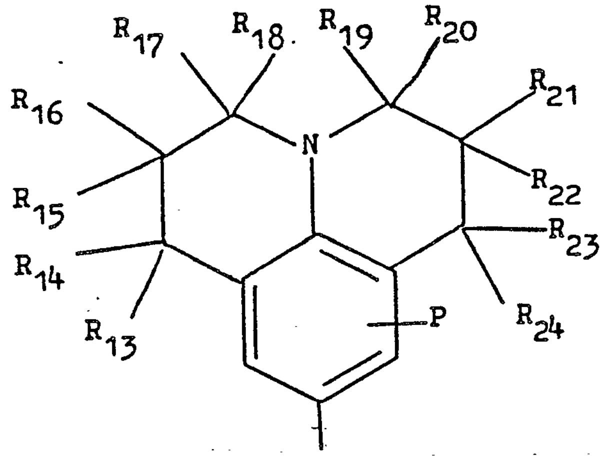

(V)

wherein P is an optional substituent having the meaning given above, and the groups $R_{13}$ to $R_{24}$ are hydrogen, alkyl, aryl or aralkyl;

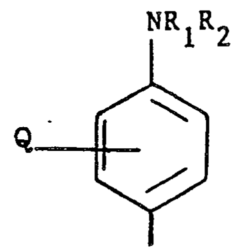

(VI)

wherein Q is hydrogen, alkyl, alkoxy, acylamino or halogen, and $R_1$ and $R_2$ are each independently hydrogen or an optionally substituted alkyl, aryl or aralkyl group or both $R_1$ and $R_2$ together with the nitrogen atom to which they are joined form a nitrogen-linked ring, provided that $R_1$ and $R_2$ are not both hydrogen at the same time; and

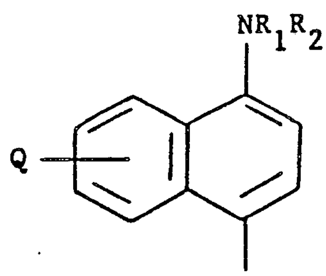

(VII)

wherein Q, and $R_1$ and $R_2$ have the meanings given above.

As optional substituents when $R_1$ and/or $R_2$ are an alkyl, aryl or aralkyl group, there may be mentioned for example —CN, —OH, alkoxy and —$NH_2$. When $R_1$ and/or $R_2$ are aryl groups optional substituents additionally include for example —$NO_2$ and alkyl.

It is preferred that neither $X^1$ nor $X^2$ are hydrogen, and for ease of manufacture, it is especially preferred that $X^1$ and $X^2$ are the same. Preferred groups $X^1$ and $X^2$ are alkyl and aryl groups. As examples of alkyl groups, there may be mentioned alkyl containing from 1 to 24, and especially from 1 to 8 carbon atoms. When both $X^1$ and $X^2$ are methyl, the compound of formula (I) is readily prepared from relatively inexpensive starting materials. As substituents which may be present in the substituted alkyl group there may be mentioned for example halogen, cyano, hydroxy, nitro and amino. As examples of aryl groups, there may be especially mentioned optionally substituted phenyl. As examples of aralkyl groups, there may be mentioned optionally substituted benzyl. As examples of alkoxy groups, there may be mentioned alkoxy groups containing from 1 to 24, and especially from 1 to 8 carbon atoms. As examples of heteroaromatic groups, which may be optionally substituted, there may be mentioned 5- or 6- membered ring nitrogen, oxygen or sulphur heteroaromatic groups, including for example pyridin-2-yl, furan-2-yl and thiophen-2-yl. As examples of carboxyalkyl groups, there may be mentioned carboxylower alkyl groups, for example

carboxymethyl. As examples of dialkylamino groups, there may be mentioned dialkylamino groups containing a total of from 2 to 24, for example from 2 to 16 carbon atoms and including dimethylamino.

The optional substituents present when $X^1$ and $X^2$ are optionally substituted aryl, aralkyl or heteroaromatic may be substituents which would be conventional to those skilled in the art. Substituents may if desired be selected to improve the solubility of the chromogenic compound in appropriate organic solvents, but it is not necessary that such substituents should be present. As examples of optional substituents there may be mentioned alkyl, especially lower alkyl, alkoxy, halogen, nitro, cyano, hydroxy, amino and alkyl or dialkyl amino.

The groups Y, P and Q will frequently be hydrogen, and very often optional substituents will not be present in groups such as alkyl, aryl, aralkyl, indolyl, carbazolyl, tetrahydroquinolyl and julolidinyl described herein. Where such optional substituents are present they will frequently be simple groups such as alkyl groups containing from 1 to 6 carbon atoms, and unsubstituted phenyl or benzyl groups. However, it should be noted that optional substituents may be present for example for convenience of manufacture. Furthermore, the presence of optional substituents may modify the properties of the unsubstituted compound in a desirable manner. Thus, for example, the presence of a higher alkyl substituent may have the effect of increasing the oil-solubility of the chromogenic compound should this be desired.

The nitrogen in the group $—NR_1R_2$ and in the heterocyclic ring system is preferably a tertiary nitrogen, and $R_1$ and $R_2$ (in formula I), $R_3$ (in formula II) and $R_5$ (in formula III) are preferably not hydrogen. $R_1$, $R_2$, $R_3$, $R_4$, $R_5$, $R_6$ and $R_{25}$ are conveniently alkyl (for example an alkyl group containing from 1 to 6 carbon atoms), benzyl or (optionally substituted) phenyl. Alternatively $R_1$ and $R_2$ and the nitrogen atom joining them together form a heterocyclic ring, for example pyrrolidinyl, morpholinyl and piperidinyl.

In general, any combination of groups $A^1$ and $A^2$ may be used, however especially preferred combinations of groups $A^1$ and $A^2$ are:

(1) indolyl (the group of formula II) combined with any one of the groups indolyl (formula II), aminophenyl (formula VI), aminonaphthyl (formula VII), carbazolyl (formula III), tetrahydroquinolyl (formula IV), dihydroquinolyl (formula IVa) and julolidinyl (formula V); and

(2) aminophenyl (the group of formula VI) combined with any one of the groups aminophenyl (formula VI), aminonaphthyl (formula VII), carbazolyl (formula III), tetrahydroquinolyl (formula IV), dihydroquinolyl (formulae IVa) and julolidinyl (formula V).

As specific examples of the chromogenic compounds of formula I there may be mentioned:—

2,3-di(4-methoxyphenyl)-7-(1-ethyl-2-methylindol-3-yl)-7-(4-N,N-diethylaminophenyl)-5,7-dihydrofuro-[3,4-b]-pyrazin-5-one;

2,3-diphenyl-7,7-di(2-acetylamino-4-N,N-diethylaminophenyl)-5,7-dihydrofuro-[3,4-b]-pyrazin-5-one;

2,3-diphenyl-7,7-di(4-N,N-diethylaminophenyl)-5,7-dihydrofuro-(3,4-b]-pyrazin-5-one;

2,3-diphenyl-7,7-di(4-N,N-dipropylaminophenyl)-5,7-dihydrofuro-[3,4-b]-pyrazin-5-one;

2,3-diethyl-7-(1-ethyl-2-methylindol-3-yl)-7-(4-N,N-diethylaminophenyl)-5,7-dihydrofuro-[3,4-b]-pyrazin-5-one;

3-phenyl-7,7-di(4-N,N-diethylaminophenyl)-5,7-dihydrofuro-[3,4-b]-pyrazin-5-one;

2,3-dimethyl-7-(1-octyl-2-methylindol-3-yl)-7-(4-N,N-diethylaminophenyl)-5,7-dihydrofuro-[3,4-b]-pyrazin-5-one;

2,3-diphenyl-7,7-di(2-methyl-4-N,N-diethylaminophenyl)-5,7-dihydrofuro-[3,4-b]-pyrazin-5-one;

2,3-diphenyl-7-(1-ethyl-2-methylindol-3-yl)-7-(4-N-ethyl, N-(2-cyanoethyl)aminophenyl)-5,7-dihydrofuro-[3,4-b]-pyrazin-5-one;

2-methyl-3-ethyl-7-(1-ethyl-2-methylindol-3-yl)-7-(4-N,N-diethylaminophenyl)-5,7-dihydrofuro-[3,4-b]-pyrazin-5-one; and

2-methyl-3-phenyl-7,7-di(4-N,N-diethylaminophenyl)-5,7-dihydrofuro-[3,4-b]-pyrazin-5-one.

The chromogenic compounds of the present invention may be prepared by known general methods.

Thus, for example, compounds of general formula (I) may be prepared by reacting a compound of general formula VIII with a compound of general formula $A^2H$ in the presence of an acid condensing agent such as phosphoric acid, sulphuric acid, zinc chloride and especially acetic anhydride.

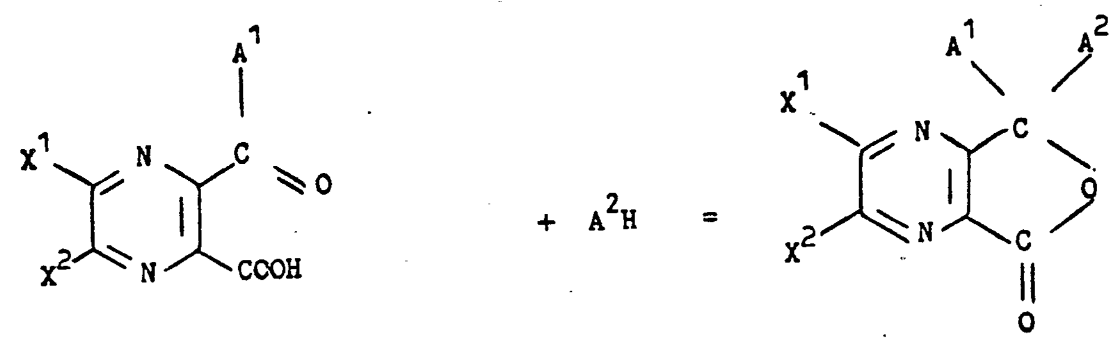

(VIII) (I)

When the reaction is complete, the reaction mass is suitably neutralised with alkali such as ammonia

and if necessary the residue may be treated with dilute mineral acid to remove any basic impurities. The product may be recovered in conventional manner, for example filtration followed by recrystallisation or chromatography.

The novel intermediate of general formula VIII may be prepared by reacting the appropriate pyrazine anhydride with a compound of formula $A^1H$:—

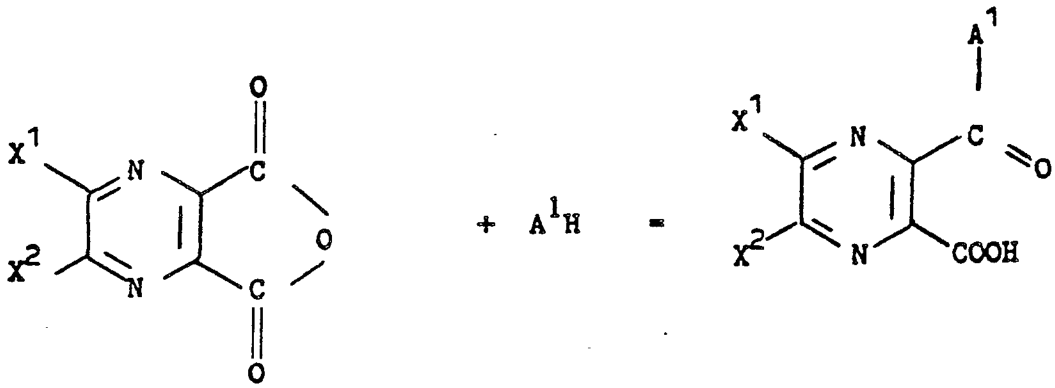

(IX) (VIII)

The reaction preferably takes place in the presence of a suitable solvent, for example toluene, xylene, methylene chloride, acetonitrile, acetic acid or chlorobenzene, and there may optionally be added a salt of a polyvalent metal, for example zinc chloride or aluminium chloride.

Compounds of formula (IX) are generally known compounds, available for example by the reaction of the appropriate diketone with 1,2-diamino-1,2-dicyanoethylene to form the corresponding dicyano pyrazine followed by hydrolysis of the cyano groups and cyclisation in the presence of for example acetic anhydride:—

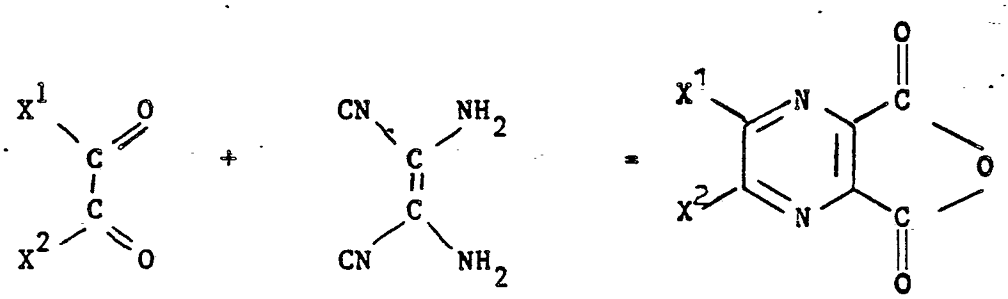

(X) (XI) (IX)

If the two groups $A^1$ and $A^2$ are the same (A), the pyrazine anhydride of formula (IX) may be reacted with two molecules of the compound AH under more forcing conditions to go directly through to the compound of formula (I).

The chromogenic compounds of the present invention, when converted into their coloured form, generally show excellent tinctorial strength, light-fastness and long-term fade resistance. These advantages tend to be combined with a relatively rapid colour development (rate of reaction with the co-reactant) and good moisture resistance giving a very effective balance of colour-forming properties. In particular, the chromogenic compounds of the present invention generally show good stability with respect to the premature development of colour (for example during encapsulation, coating and storage of the coated paper) and generally give colourless emulsions and derived coated papers.

The chromogenic compounds of the present invention may be used in conventional manner. They may be used in conventional solvents known in the art such as alkylated biphenyls (for example monoisopropyl biphenyl), naphthalenes (for example partially hydrogenated naphthalene), terphenyls (which may be partially or totally hydrogenated), di esters of dicarboxylic acids (for example alkyl oxalates and phthalates) and polyhalogenated paraffins. The solution in a suitable organic solvent may be micro-encapsulated using conventional techniques, and the encapsulated system incorporated into pressure-sensitive or thermal copying materials.

Thus, the capsules walls can be formed evenly around the droplets of the colour former solution by coacervation; and the encapsulating material can consist of gelatin and gum arabic, as described e.g. in US patent 2800457. The capsules can also be formed from an aminoplast or a modified aminoplast by

polycondensation, as described in British patent specifications 989264, 1156725, 1301052 and 1355124. Other suitable microcapsules are formed by interfacial polymerisation e.g. capsules formed from polyester, polycarbonate, polysulphonamide, polysulphonate, but in particular from polyamide or polyurethane.

The microcapsules containing the compounds of formula (I) can be used for the production of a wide variety of known kinds of pressure-sensitive copying material. The various systems differ substantially from one another in the arrangement of the capsules, of the colour reactions, and of the support. A preferred arrangement is that in which the encapsulated chromogenic compound is in the form of a layer on the back of a transfer sheet and the developer is in the form of a layer on the face of a receiver sheet.

Another arrangement of the components is that wherein the microcapsules which contain the chromogenic compounds, and the developer, are in or on the same sheet, in the form of one or more individual layers, or are present in the paper pulp.

Conventional co-reactants may be used for the chromogenic compounds of the present invention and as examples there may be mentioned active clays, (for example acid clay, attapulgite, zeolite or bentonite); a solid organic acid (such as succinic acid, tannic acid or benzoic acid); an acidic polymer (such as phenolformaldehyde, phenol-acetylene polymer, residual acid group-containing styrene-maleic anhydride polymer or salicylic acid-formaldehyde polymer); and heavy metal salicylates (such as zinc salicylate). Bisphenol A is commonly used as a co-reactant in heat-sensitive applications.

The invention is illustrated by the following Examples in which all parts and percentages are by weight unless otherwise stated.

Example 1

Preparation of 2,3-diphenyl-7-(1-ethyl-2-methylindol-3-yl)-7-(2-methoxy-4-N,N-diethylaminophenyl)-5,7-dihydrofuro[3,4-b]-pyrazine-5-one (XIII).

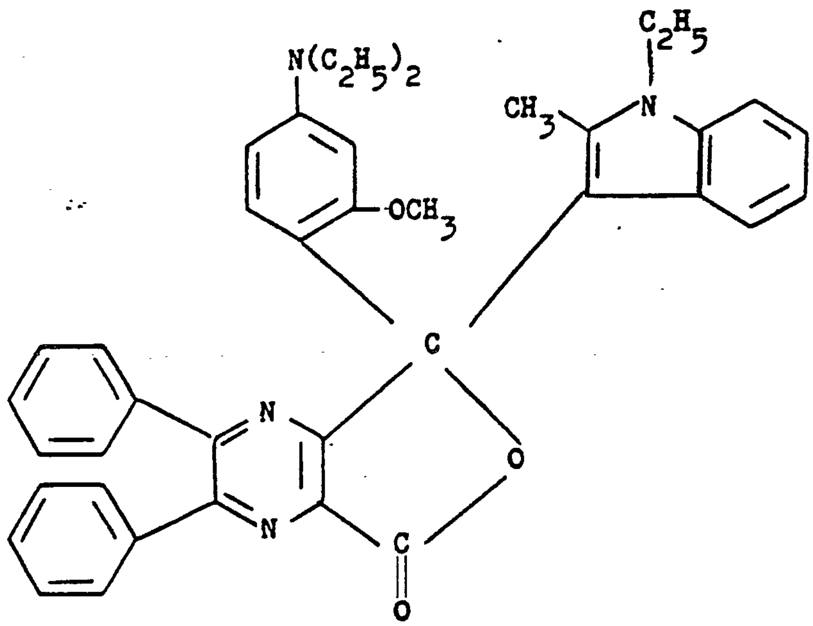

(XII)

A mixture of 5,6-diphenylpyrazine-2,3-dicarboxylic anhydride (4 g), 1-ethyl-2-methylindole (2.1 g) and acetonitrile (20 ml) was stirred at ambient temperature for 16 hours. The product was isolated by filtration, washed with methanol and dried to yield (1-ethyl-2-methylindol-3-yl)-(2-carboxy-5,6-diphenylpyrazin-3-yl) ketone (4 g).

A mixture of the above keto acid (1 g), 3-methoxy-N,N-diethylaniline (0.4 g) and acetic anhydride (10 ml) was stirred at 90°C for 2 hours. After cooling, the solution was treated with 15% aqueous ammonia (60 ml), stirred 1 hour and the tarry product was isolated by decantation. After treatment with dilute hydrochloric acid the solid product was isolated by filtration, washed with water and dried to yield compound (XII) (1.1 g).

A 1% solution of the product in a polyhalogenated paraffins solvent showed little or no colour formation when applied to base paper.

$\lambda_{max}$ = 595 nm.

Examples 2—22

Examples 2—22 shown in Table 1 can be prepared by the method of Example 1. The compounds have the general formula:

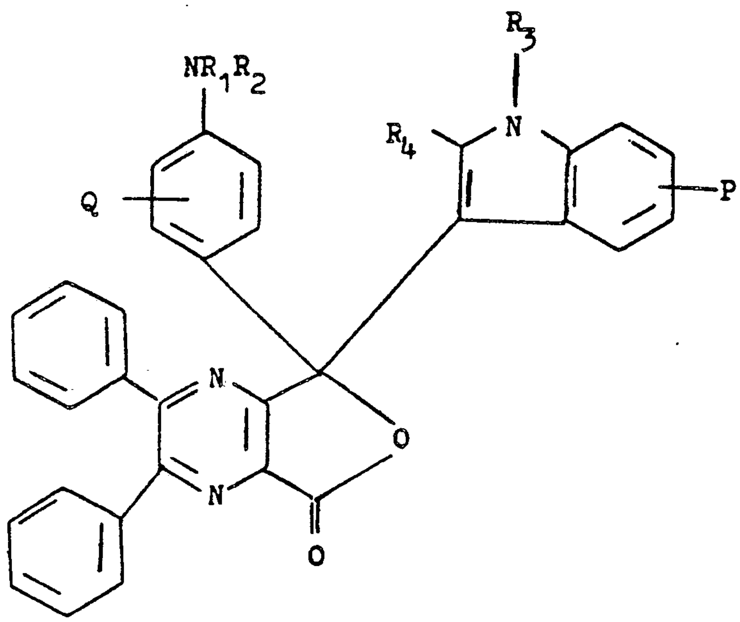

and are identifiable in Table 1 by the substituents.

Table 1

| Ex. | Q | $R_1$ | $R_2$ | P | $R_3$ | $R_4$ | λmax. |
|---|---|---|---|---|---|---|---|
| 2 | H | $nC_3H_7$ | $nC_3H_7$ | H | $C_2H_5$ | $CH_3$ | 610 |
| 3 | H | $CH_3$ | $CH_3$ | H | H | " | 598 |
| 4 | H | " | " | H | $nC_4H_9$ | " | 608 |
| 5 | H | $C_2H_5$ | $C_2H_5$ | H | $C_2H_5$ | " | 610 |
| 6 | H | $nC_4H_9$ | $nC_4H_9$ | H | " | " | 610 |
| 7 | H | $nC_3H_7$ | $nC_3H_7$ | H | H | " | 604 |
| 8 | H | $C_2H_5$ | $CH_2C_6H_5$ | H | $C_2H_5$ | " | 611 |
| 9 | H | " | $CH_2CH_2CN$ | H | " | " | 611 |
| 10 | H | $CH_2CH_2OCOCH_3$ | $CH_2CH_2OCOCH_3$ | H | " | " | 608 |
| 11 | H | $CH_3$ | $C_2H_5$ | H | " | " | 610 |
| 12 | H | $C_2H_5$ | " | H | $nC_8H_{17}$ | " | 611 |
| 13 | H | " | " | H | $C_2H_5$ | $C_6H_5$ | 615 |
| 14 | H | " | " | H | $CH_2C_6H_5$ | $CH_3$ | 610 |
| 15 | H | $CH_3$ | $CH^3$ | $CH_3$ | $C_2H_5$ | " | 610 |
| 16 | $OCH_3$ | $C_2H_5$ | $C_2H_5$ | H | $nC_4H_9$ | " | 595 |
| 17 | $OC_2H_5$ | " | " | H | $C_2H_5$ | " | 598 |
| 18 | $OC_3H_7$ | " | " | H | " | " | 598 |
| 19 | $OC_4H_9$ | " | " | H | " | " | 600 |
| 20 | $NHCOCH_3$ | " | " | H | " | " | 625 |
| 21 | Cl | " | " | H | " | " | 615 |
| 22 | $CH_3$ | " | " | H | " | " | 615 |

Example 23

Preparation of 2,3-dimethyl-7-(1-ethyl-2-methylindol-3-yl)-7-(4-N,N-diethylaminophenyl)-5,7-dihydrofuro[3,4-b]-pyrazin-5-one (XIII).

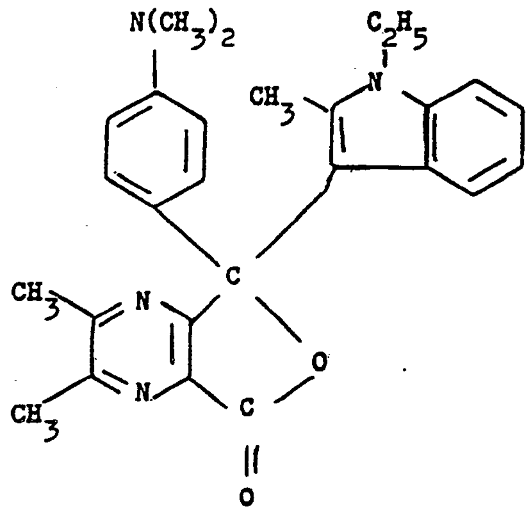

(XIII)

(1-ethyl-2-methylindol-3-yl)-(2-carboxy-5,6-dimethylpyrazin-3-yl)ketone was prepared by the method of Example 1 using 5,6-dimethylpyrazine-2,3-dicarboxylic anhydride as starting material.

A mixture of the above keto-acid (0.5 g), N,N-dimethylaniline (0.2 g) and acetic anhydride (6 ml) was stirred at 90°C for 4 hours. After cooling to room temperature the solution was treated with 15% aqueous ammonia (50 ml), stirred for 1 hour and the tarry product was purified by column chromatography to yield compound (XIII).

A 1% solution of the product in monoisopropyl biphenyl showed little or no colour formation when applied to base paper.

$\lambda_{max}$ 602 nm.

Examples 24—59

Examples 24—59 shown in Table 2 can be prepared by the method of Example 23. The compounds have the general formula:

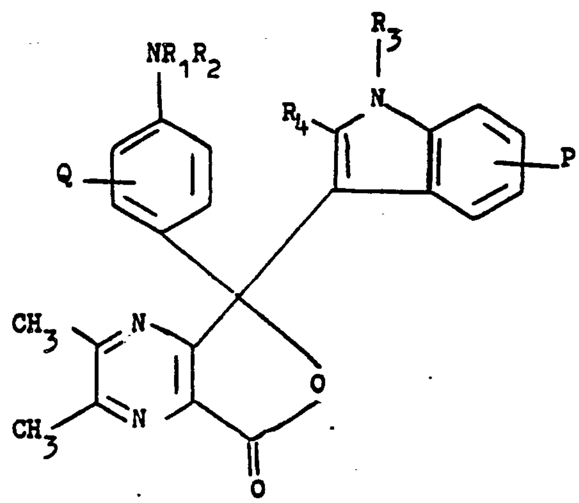

and are identified in Table 2 by the substituents.

Table 2

| Ex. | Q | $R_1$ | $R_2$ | P | $R_3$ | $R_4$ | $\lambda$max. |
|---|---|---|---|---|---|---|---|
| 24 | H | $C_2H_5$ | $C_2H_5$ | H | $C_2H_5$ | $CH_3$ | 602 |
| 25 | H | $nC_3H_7$ | $nC_3H_7$ | H | " | " | 602 |
| 26 | H | $nC_4H_9$ | $nC_4H_9$ | H | " | " | 602 |

Table 2 (continued)

| Ex. | Q | $R_1$ | $R_2$ | P | $R_3$ | $R_4$ | λmax. |
|---|---|---|---|---|---|---|---|
| 27 | H | $C_2H_5$ | $CH_2C_6H_5$ | H | " | " | 605 |
| 28 | H | " | $C_2H_4CN$ | H | " | " | 605 |
| 29 | H | $CH_2CH_2OCOCH_3$ | $CH_2CH_2OCOCH_3$ | H | " | " | 605 |
| 30 | $CH_3$ | $C_2H_5$ | $C_2H_5$ | H | " | " | 610 |
| 31 | $OCH_3$ | " | " | H | " | " | 590 |
| 32 | $OC_2H_5$ | " | " | H | " | " | 590 |
| 33 | $OC_3H_5$ | " | " | H | " | " | 593 |
| 34 | $OC_4H_5$ | " | " | H | " | " | 593 |
| 35 | $NHCOCH_3$ | " | " | H | " | " | 615 |
| 36 | Cl | $CH_3$ | $CH_3$ | H | " | " | 600 |
| 37 | H | " | $C_2H_5$ | H | " | " | 602 |
| 38 | H | $nC_4H_9$ | $nC_4H_9$ | H | H | " | 590 |
| 39 | H | $C_2H_5$ | $C_2H_5$ | H | $nC_3H_7$ | " | 602 |
| 40 | H | " | " | H | $nC_4H_9$ | " | 602 |
| 41 | H | " | " | H | $CH_3$ | " | 600 |
| 42 | H | " | " | H | $C_8H_{17}$ | " | 603 |
| 43 | H | $C_3H_7$ | $C_3H_7$ | H | $C_2H_5$ | $C_6H_5$ | 610 |
| 44 | H | $CH_3$ | $CH_3$ | H | $CH_2C_6H_5$ | $CH_3$ | 608 |
| 45 | H | $C_2H_5$ | $C_2H_5$ | $CH_3$ | $C_2H_5$ | " | 602 |
| 46 | H | $CH_3$ | $CH_3$ | H | $nC_8H_{17}$ | " | 603 |
| 47 | H | $C_2H_5$ | $CH_2C_6H_5$ | H | " | " | 606 |
| 48 | H | " | " | H | $nC_4H_9$ | " | 606 |
| 49 | H | $nC_3H_7$ | $nC_3H_7$ | H | $nC_8H_{17}$ | $CH_3$ | 606 |
| 50 | H | $nC_4H_9$ | $nC_4H_9$ | H | " | " | 605 |
| 51 | H | " | " | H | $nC_4H_9$ | " | 603 |
| 52 | H | $nC_5H_{11}$ | $nC_5H_{11}$ | H | " | " | 606 |
| 53 | H | $nC_6H_{13}$ | $nC_6H_{13}$ | H | " | " | 606 |
| 54 | H | " | " | H | $C_2H_5$ | " | 602 |
| 55 | H | $CH_3$ | $CH_3$ | H | $nC_6H_{13}$ | " | 604 |
| 56 | H | $C_2H_5$ | $C_2H_5$ | H | " | " | 606 |
| 57 | H | $nC_3H_7$ | $nC_3H_7$ | H | " | " | 606 |
| 58 | H | $nC_4H_9$ | $nC_4H_9$ | H | " | " | 605 |
| 59 | H | $nC_5H_{11}$ | $nC_5H_{11}$ | H | " | " | 606 |

Example 60

Preparation of 2,3-diphenyl-7-(1-ethyl-2-methylindol-3-yl)-7-(4-N,N-dimethylaminophenyl)-5,7-dihydrofuro[3,4-b]-pyrazin-5-one (XIV).

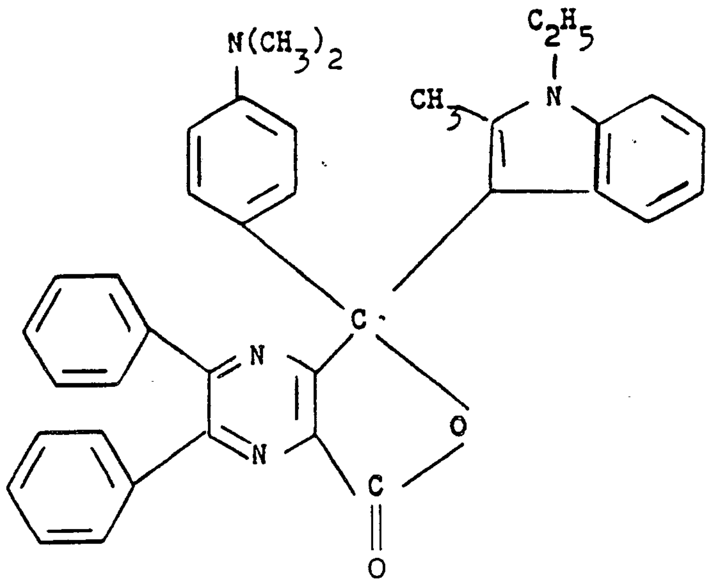

(XIV)

N,N-dimethylaniline (15 g) and then anhydrous aluminium chloride (15 g) were added slowly to ice-cooled methylene chloride (160 ml), the temperature was allowed to rise to room temperature and 5,6-diphenylpyrazine-2,3-dicarboxylic anhydride (28.7 g) was added gradually. The reaction mixture was stirred under reflux for 72 hours, cooled, steam distilled and the residue was isolated by filtration. After dissolving in aqueous ammonia and screening the product, (4-N,N-dimethylaminophenyl)-(2-carboxy-5,6-diphenylpyrazin-3-yl) ketone (7.5 g) was precipitated by the addition of dilute hydrochloric acid.

A mixture of the above keto-acid (1.0 g), 1-ethyl-2-methylindole (0.4 g) and acetic anhydride (10 ml) was stirred at 95°C for 2 hours. After cooling to room temperature the solution was treated with 15% aqueous ammonia (60 ml), stirred for 2 hours. The product was isolated by filtration and washed well with water to yield compound (XIV) (1.2 g).

A 1% solution of the product in monoisopropyl biphenyl showed little or no colour formation when applied to base paper.

$\lambda_{max}$ = 610 nm.

Example 61

Preparation of 2,3-dimethyl-7-(4-N,N-dimethylaminophenyl)-7-(4-N,N-dipropylaminophenyl)-5,7-dihydrofuro [3,4-b] pyrazin-5-one (XV).

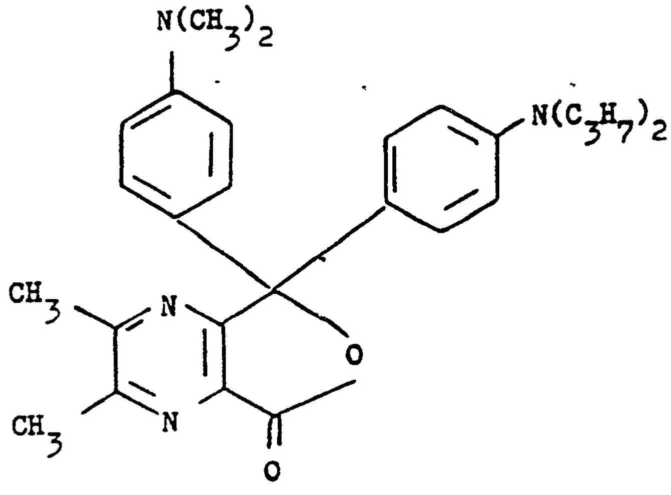

(XV)

(4-N,N-dimethylaminophenyl)-(2-carboxy-5,6-dimethylpyrazin-3-yl)ketone was prepared by the method of Example 60 using 5,6-dimethylpyrazin-2,3-dicarboxylic anhydride as starting material.

A mixture of the above keto acid (1.0 g), N,N-di-n-propylaniline (0.6 g) and acetic anhydride (10 ml) was

stirred at 90°C for 3 hours. After cooling to room temperature, the solution was treated with 15% aqueous ammonia (80 ml) and stirred for 3 hours. The product was isolated by filtration and purified by chromatography to yield compound (XV). A 1% solution of the product in polyhalogenated paraffins showed little or no colour formation when applied to base paper.

$\lambda_{max}$ = 643 nm.

Examples 62—93

Examples 62—93 shown in Table 3 can be prepared by the general method of Example 61. The compounds have the general formula:

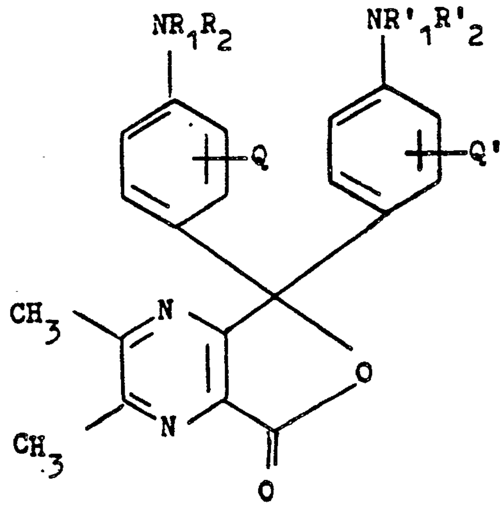

and are identified in Table 3 by the substituents.

**Table 3**

| Ex. | Q | $R_1$ | $R_2$ | $Q^1$ | $R'_1$ | $R'_2$ | λmax. |
|---|---|---|---|---|---|---|---|
| 62 | H | $CH_3$ | $CH_3$ | H | $CH_3$ | $CH_3$ | 640 |
| 63 | H | $C_2H_5$ | $C_2H_5$ | H | $C_2H_5$ | $C_2H_5$ | 643 |
| 64 | H | $nC_3H_7$ | $nC_3H_7$ | H | $nC_3H_7$ | $nC_3H_7$ | 652 |
| 65 | H | $nC_4H_9$ | $nC_4H_9$ | H | $nC_4H_9$ | $nC_4H_9$ | 652 |
| 66 | H | $C_2H_5$ | $CH_2C_6H_5$ | H | $C_2H_5$ | as $R_2$ | 647 |
| 67 | H | " | $CH_2CH_2CN$ | H | " | as $R_2$ | 640 |
| 68 | H | " | $CH_2CH_2OCOCH_3$ | H | " | as $R_2$ | 640 |
| 69 | H | $CH_3$ | $nC_4H_9$ | H | $CH_3$ | $nC_4H_9$ | 643 |
| 70 | $CH_3$ | $C_2H_5$ | $C_2H_5$ | $CH_3$ | $C_2H_5$ | $C_2H_5$ | 670 |
| 71 | $CH_3$ | " | $CH_2C_6H_5$ | $CH_3$ | " | as $R_2$ | 668 |
| 72 | $NHCOCH_3$ | " | $C_2H_5$ | $NHCOCH_3$ | $C_2H_5$ | $C_2H_5$ | 668 |
| 73 | Cl | " | " | Cl | " | " | 660 |
| 74 | H | $CH_3$ | $CH_3$ | H | " | " | 641 |
| 75 | H | " | " | H | $nC_3H_7$ | $nC_3H_7$ | 643 |

Table 3 (continued)

| Ex. | Q | $R_1$ | $R_2$ | $Q^1$ | $R'_1$ | $R'_2$ | λmax. |
|---|---|---|---|---|---|---|---|
| 76 | H | " | " | H | $nC_4H_9$ | $nC_4H_9$ | 643 |
| 77 | H | $C_2H_5$ | $C_2H_5$ | H | $nC_3H_7$ | $nC_3H_7$ | 643 |
| 78 | H | " | " | H | $nC_4H_9$ | $nC_4H_9$ | 643 |
| 79 | H | $CH_3$ | $CH_3$ | $CH_3$ | $C_2H_5$ | $C_2H_5$ | 650 |
| 80 | H | " | " | $NHCOCH_3$ | " | " | 660 |
| 81 | H | " | " | $OCH_3$ | " | " | 640 |
| 82 | H | " | " | $OC_3H_7$ | " | " | 640 |
| 83 | H | $CH_3$ | $CH_3$ | $OC_2H_5$ | $C_2H_5$ | $C_2H_5$ | 639 |
| 84 | H | $C_2H_5$ | $C_2H_5$ | " | " | " | 639 |
| 85 | $CH_3$ | " | " | $CH_3$ | " | $CH_2CH_2CN$ | 660 |
| 86 | H | $CH_3$ | $CH_3$ | H | " | " | 640 |
| 87 | H | " | " | H | " | $CH_2C_6H_5$ | 642 |
| 88 | H | " | " | H | " | $CH_2CH_2OCOCH_2$ | 640 |
| 89 | H | $nC_3H_7$ | $nC_3H_7$ | $CH_3$ | " | $C_2H_5$ | 650 |
| 90 | H | $C_2H_5$ | $C_2H_5$ | H | " | $CH_2C_6H_5$ | 642 |
| 91 | H | $CH_3$ | $CH_3$ | H | " | " | 642 |
| 92 | H | " | " | $CH_3$ | " | " | 655 |
| 93 | H | $C_2H_5$ | $C_2H_5$ | $CH_3$ | " | " | 655 |

Example 94

Preparation of 2,3-diphenyl-7,7-di(4-N,N-dimethylaminophenyl)-5-7-dihydrofuro[3,4-b]pyrazin-5-one (XVI).

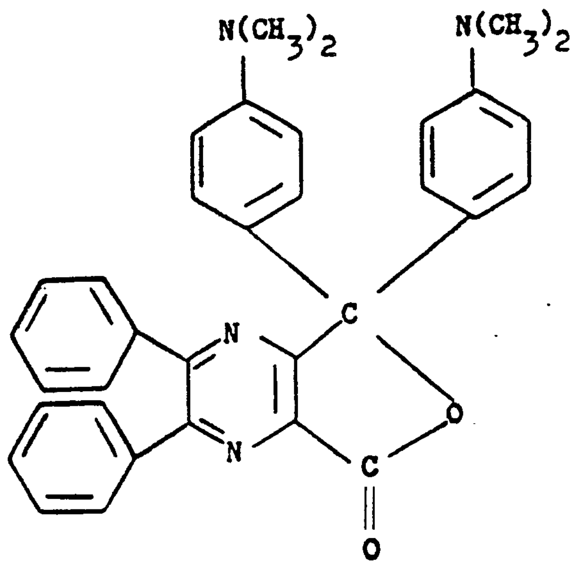

(XVI)

A mixture of the keto-acid of Example 60 (1.0 g), N,N-dimethylaniline (0.3 g) and acetic anhydride (16

ml) was stirred at 90°C for 3 hours. After cooling to room temperature the product was isolated by filtration, washed with ethanol and dried to yield compound (XVI) (1.0 g).

A 1% solution of the product in polyhalogenated paraffins showed little or no colour formation on base paper.

$\lambda_{max}$ = 650 nm.

Examples 95—121

Examples 95—121 shown in Table 4 can be prepared by the method of Example 94. The compounds have the general formula:

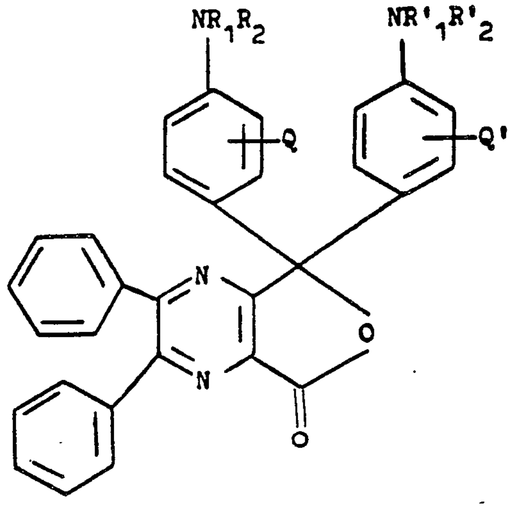

and are identified in Table 4 by the substituents.

Table 4

| Ex. | Q | $R_1$ | $R_2$ | $Q^1$ | $R'_1$ | $R'_2$ | λmax. |
|---|---|---|---|---|---|---|---|
| 95 | H | $C_2H_5$ | $C_2H_5$ | H | $C_2H_5$ | $C_2H_5$ | 650 |
| 96 | H | $nC_3H_7$ | $nC_3H_7$ | H | $nC_3H_7$ | $nC_3H_7$ | 655 |
| 97 | H | $nC_4H_9$ | $nC_4H_9$ | H | $nC_4H_9$ | $nC_4H_9$ | 658 |
| 98 | H | $C_2H_5$ | $CH_2C_6H_5$ | H | $C_2H_5$ | as $R_2$ | 650 |
| 99 | H | " | $CH_2CH_2CN$ | H | " | as $R_2$ | 650 |
| 100 | H | " | $CH_2CH_2OCOCH_3$ | H | " | as $R_2$ | 648 |
| 101 | H | $CH_3$ | $nC_4H_9$ | H | $CH_3$ | $nC_4H_9$ | 655 |
| 102 | $CH_3$ | $C_2H_5$ | $C_2H_5$ | $CH_3$ | $C_2H_5$ | $C_2H_5$ | 670 |
| 103 | $CH_3$ | " | $CH_2C_6H_5$ | $CH_3$ | " | as $R_2$ | 672 |
| 104 | $NHCOCH_3$ | " | $C_2H_5$ | $NHCOCH_3$ | " | $C_2H_5$ | 670 |
| 105 | Cl | " | " | Cl | " | " | 665 |
| 106 | H | $CH_3$ | $CH_3$ | H | " | " | 650 |
| 107 | H | " | " | H | $nC_3H_7$ | $nC_3H_7$ | 653 |
| 108 | H | " | " | H | $nC_4H_9$ | $nC_4H_9$ | 655 |
| 109 | H | $C_2H_5$ | $C_2H_5$ | H | $nC_3H_7$ | $nC_3H_7$ | 655 |
| 110 | H | " | " | H | $nC_4H_9$ | $nC_4H_9$ | 655 |

**Table 4**

| Ex. | Q | $R_1$ | $R_2$ | $Q^1$ | $R'_1$ | $R'_2$ | λmax. |
|---|---|---|---|---|---|---|---|
| 111 | H | $CH_3$ | $CH_3$ | $CH_3$ | $C_2H_5$ | $C_2H_5$ | 665 |
| 112 | H | " | " | $NHCOCH_3$ | " | " | 660 |
| 113 | H | " | " | $OCH_3$ | " | " | 645 |
| 114 | H | " | " | $OC_3H_7$ | " | " | 645 |
| 115 | H | " | " | $OC_2H_5$ | " | " | 646 |
| 116 | H | $C_2H_5$ | $C_2H_5$ | " | " | " | 646 |
| 117 | $CH_3$ | " | " | $OCH_3$ | " | " | 650 |
| 118 | $CH_3$ | " | " | $CH_3$ | " | $CH_2CH_2CN$ | 666 |
| 119 | H | $CH_3$ | $CH_3$ | H | " | " | 641 |
| 120 | H | " | " | H | " | $CH_2C_6H_5$ | 646 |
| 121 | H | " | " | H | " | $CH_2CH_2OCOCH_3$ | 646 |

Example 122

Preparation of 2,3-diphenyl-7,7-di(1-ethyl-2-methylindol-3-yl)-5-7-dihydrofuro[3,4-b]pyrazin-5-one (XVII).

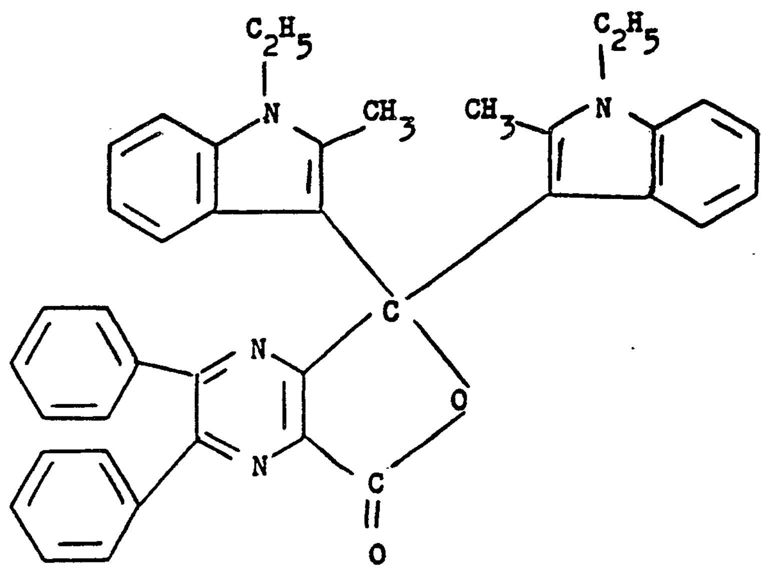

(XVII)

A mixture of keto-acid of Example 1 (1.0 g), 1-ethyl-2-methylindole and acetic anhydride (10 ml) was stirred at 75°C for 2 hours. After cooling to room temperature the mixture was treated with 15% aqueous ammonia (60 ml), stirred for 2 hours, the product was isolated by filtration and washed with water to yield Compound (XVII).

A 1% solution of the product in polyhalogenated paraffins showed little or no colouration in contact with base paper.

$\lambda_{max}$ = 562 nm.

Examples 123—135

Examples 123—135 shown in Table 5 can be prepared by the method of Example 122. The compounds have the general formula:

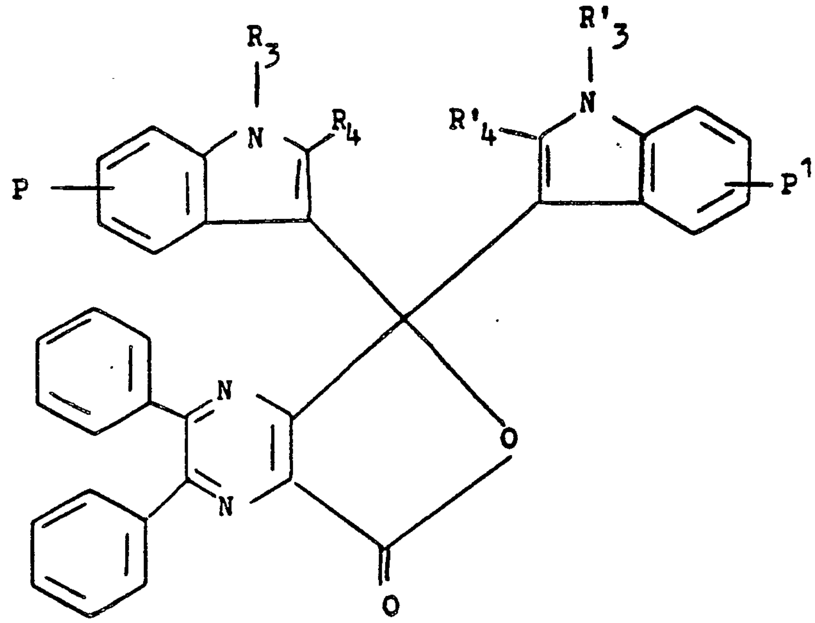

and are identified in Table 5 by the substituents.

Table 5

| Ex. | P | $R_3$ | $R_4$ | $P^1$ | $R'_3$ | $R'_4$ | λmax. |
|---|---|---|---|---|---|---|---|
| 123 | H | $C_2H_5$ | $CH_3$ | H | $CH_3$ | $CH_3$ | 560 |
| 124 | H | " | " | H | $nC_3H_7$ | " | 562 |
| 125 | H | " | " | H | $nC_4H_9$ | " | 565 |
| 126 | H | " | " | H | $nC_8H_{17}$ | " | 570 |
| 127 | H | $nC_3H_7$ | " | H | $nC_3H_7$ | " | 568 |
| 128 | H | $nC_4H_9$ | " | H | $nC_4H_9$ | " | 570 |
| 129 | H | $nC_8H_{17}$ | " | H | $nC_8H_{17}$ | " | 572 |
| 130 | H | $C_2H_5$ | " | H | $C_2H_5$ | $C_6H_5$ | 575 |
| 131 | H | " | " | $CH_3$ | " | $CH_3$ | 560 |
| 132 | $CH_3$ | " | " | $CH_3$ | " | " | 561 |
| 133 | H | $CH_3$ | " | H | $CH_3$ | " | 560 |
| 134 | H | $CH_2C_6H_5$ | " | H | $CH_2C_6H_5$ | " | 565 |
| 135 | H | " | " | H | $C_2H_5$ | " | 562 |

Example 136

Preparation of 2,3-dimethyl-7,7-di(1-ethyl-2-methylindol-3-yl)-5-7-dihydrofuro[3,4-b]pyrazin-5-one (XVIII).

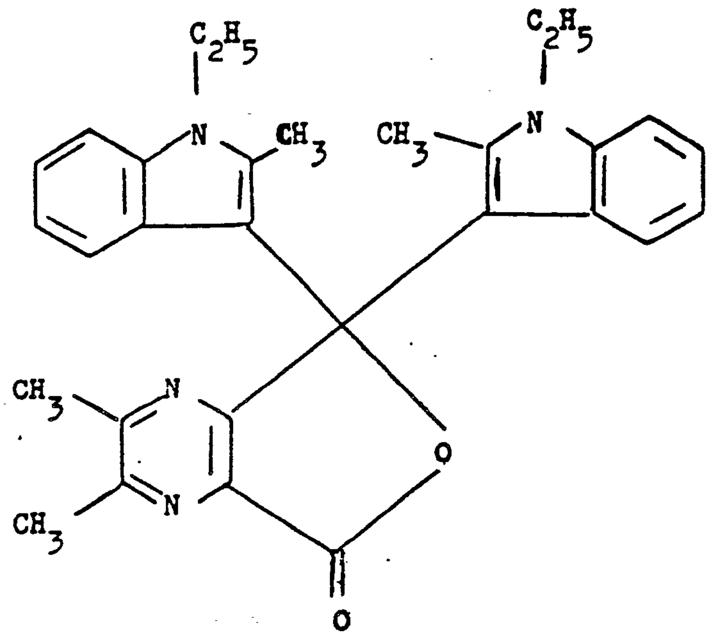

(XVIII)

A mixture of the keto acid of Example 2 (1.0 g), 1-ethyl-2-methylindole (0.5 g) and acetic anhydride (10 ml) was stirred at 75°C for 1½ hours. After cooling to room temperature, the mixture was treated with 15% aqueous ammonia (100 ml) and stirred 4 hours. The product was isolated by filtration and purified by column chromatography to yield compound (XVIII).

A 1% solution of the product in a dialkyl naphthalene solvent showed little or no colouration on base paper.

Examples 137—150

Examples 137—150 shown in Table 6 can be prepared by the method of Example 136. The compounds have the general formula:

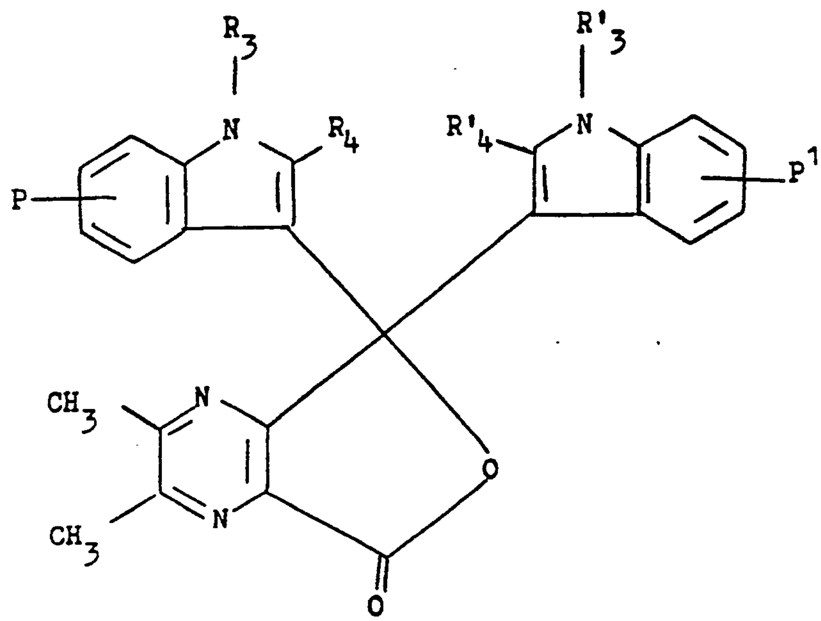

and are identified in Table 6 by the substituents.

Table 6

| Ex. | P | $R_3$ | $R_4$ | $P^1$ | $R'_3$ | $R'_4$ | λmax. |
|---|---|---|---|---|---|---|---|
| 137 | H | $C_2H_5$ | $CH_3$ | H | $CH_3$ | $CH_3$ | 553 |
| 138 | H | " | " | H | $nC_3H_7$ | " | 555 |
| 139 | H | " | " | H | $nC_4H_9$ | " | 555 |
| 140 | H | " | " | H | $nC_8H_{17}$ | " | 560 |
| 141 | H | " | " | H | $C_2H_5$ | " | 553 |
| 142 | H | $nC_3H_7$ | " | H | $nC_3H_7$ | " | 555 |
| 143 | H | $nC_4H_9$ | " | H | $nC_4H_9$ | " | 557 |
| 144 | H | $nC_8H_{17}$ | " | H | $nC_8H_{17}$ | " | 560 |
| 145 | H | $CH_3$ | " | H | $CH_3$ | " | 550 |
| 146 | H | $C_2H_5$ | " | H | $C_2H_5$ | $C_6H_5$ | 555 |
| 147 | H | " | " | H | $CH_2C_6H_5$ | $CH_3$ | 552 |
| 148 | H | " | " | $CH_3$ | $C_2H_5$ | " | 553 |
| 149 | $CH_3$ | " | " | $CH_3$ | " | " | 554 |
| 150 | H | $CH_2C_6H_5$ | " | H | $CH_2C_6H_5$ | " | 552 |

Example 151

Preparation of 2,3-difuryl-7-(1-ethyl-2-methylindol-3-yl)-7-(4-N,N-diethylaminophenyl)-5,7-dihydrofuro[3,4-b]pyrazin-5-one (XIX).

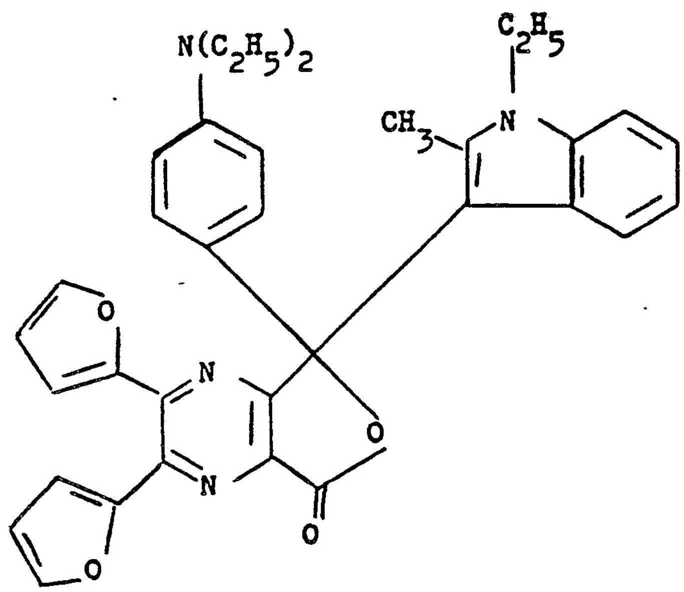

(XIX)

(1-ethyl-2-methylindol-3-yl)-(2-carboxy-5,6-difurylpyrazin-3-yl)-ketone was prepared by the method of Example 1 using 5,6-difurylpyrazine-2,3-dicarboxylic anhydride as starting material.

A mixture of the above keto acid (1.0 g), N,N-diethylaniline (0.4 g) and acetic anhydride (10 ml) was stirred at 90°C for 3 hours. After cooling to room temperature, the solution was treated with 15% aqueous ammonia (150 ml) and stirred for 2 hours. The tarry product was isolated by decantation and was purified by chromatography to yield compound XIX.

A 1% solution of the product in a dialkyl naphthalene solvent showed little or no colouration on base paper.

$\lambda_{max}$ = 610 nm.

Examples 152—162

Examples 152—162 shown in Table 7 can be prepared by the method of Example 151. The compounds have the general formula:

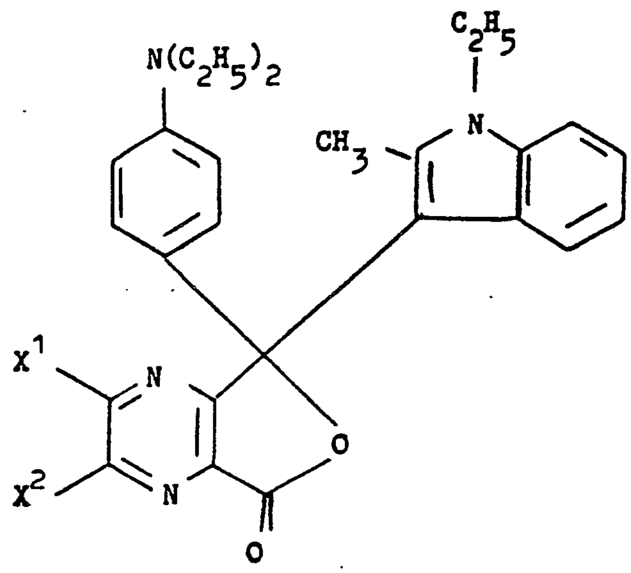

and are identified in Table 7 by the substituents.

Table 7

| Example | $X^1$ | $X^2$ | max |
|---|---|---|---|
| 152 | 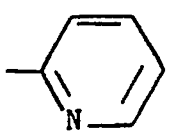 | 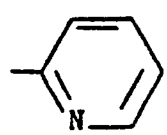 | 608 |
| 153 | 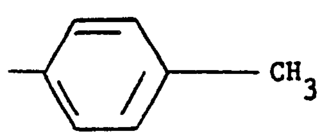 | 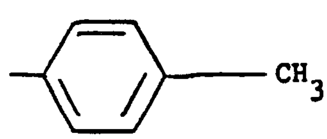 | 610 |
| 154 | 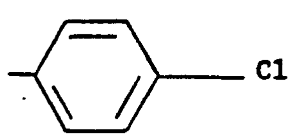 | 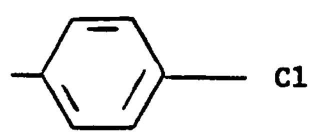 | 612 |
| 155 | 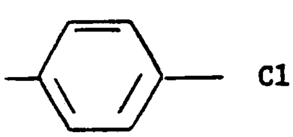 | 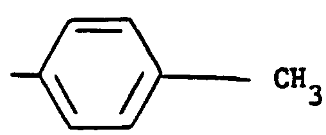 | 611 |

Table 7

| Example | X1 | X2 | max |
|---|---|---|---|
| 156 | $CH_3$ | $nC_4H_9$ | 602 |
| 157 | $nC_4H_9$ | " | 603 |
| 158 | $CH_3$ | $isoC_3H_7$ | 605 |
| 159 | $CH_2Br$ | $CH_2Br$ | 610 |
| 160 | $CH_3$ | $nC_3H_7$ | 605 |
| 161 | " | 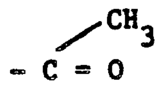 | 611 |
| 162 | 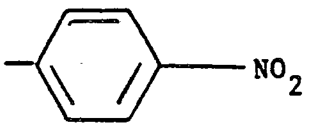 | 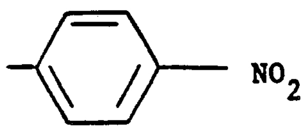 | 615 |

Example 163

Preparation of 2,3-diphenyl-7,7-di(2-methoxy-4-N,N-diethylaminophenyl)-5,7-dihydrofuro[3,4-b]pyrazin-5-one (XX).

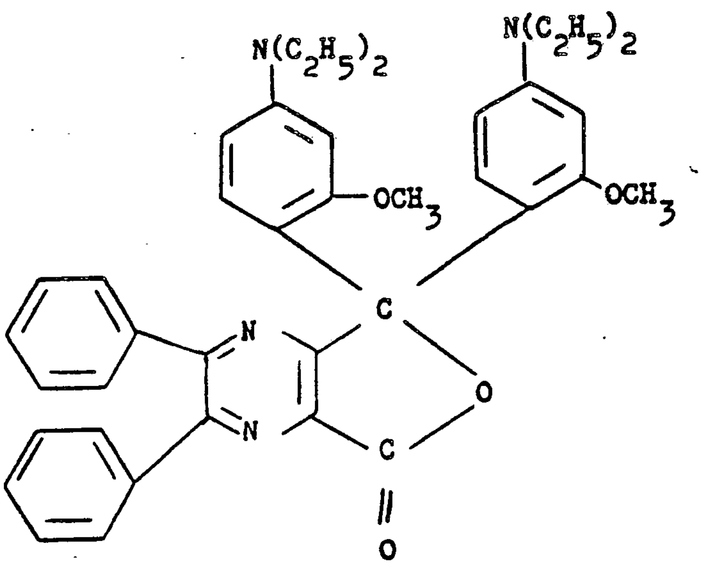

(XX)

A mixture of 5,6-diphenylpyrazine-2,3-dicarboxylic anhyridride (0.5 g), 3-methoxy-N,N-diethylaniline (0.6 g) and acetic anhydride (7.5 ml) was stirred at 85°C for 4½ hours. After cooling to room temperature, the solution was treated with 5% aqueous ammonia (60 ml), stirred for 1 hour and the tarry product was isolated by decantation. After treatment with dilute hydrochloric acid the solid product was isolated by filtration, washed with water and dried to yield compound (XX) (0.5 g).

A 1% solution of the product in polyhalogenated paraffins showed little or no colour formation on base paper.

By way of comparison a 1% solution in the same solvent of a corresponding compound having no substituents on the pyrazine ring showed extensive and commercially undesirable colour formation when applied in the same manner to base paper.

$\lambda_{max}$ = 641 nm.

Example 164

Preparation of 2,3-dimethyl-7,7-di(2-methoxy-4-N,N-diethylaminophenyl)-5,7-dihydrofuro[3,4-b]pyrazin-5-one (XXI).

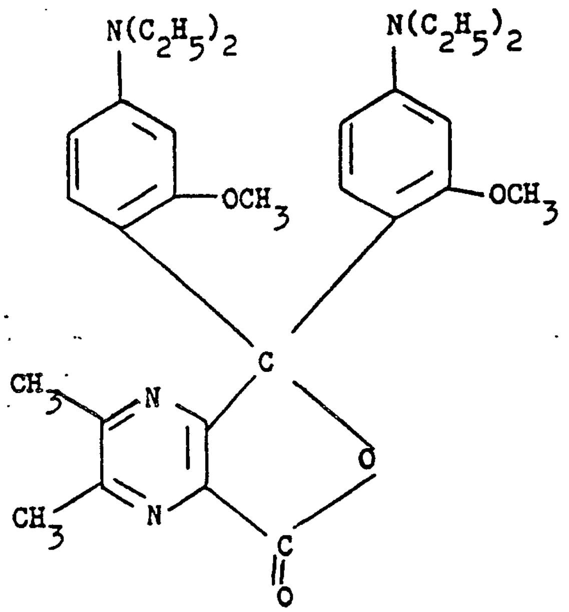

(XXI)

A mixture of 5,6-dimethylpyrazine-2,3-dicarboxylic anhydride (1 g), 3-methoxy-N,N-diethylaniline (2 g) and acetic anhydride (18 ml) was stirred at 85°C for 4 hours. After cooling to room temperature, the solution was treated with 15% aqueous ammonia (100 ml), stirred for 1 hour and the tarry product was isolated by decantation. After treatment with dilute hydrochloric acid, the solid was isolated by filtration, washed with water to yield compound (XXI).

A 1% solution of the product in polyhalogenated paraffins showed little or no colour formation when applied to base paper.

$\lambda_{max}$ = 632 nm.

Example 165

Preparation of 2,3-diphenyl-7-(4-N,N-dimethylaminophenyl)-7-(4-methylanilino)naphth-1-yl)-5,7-dihydrofuro[3,4-b]-pyrazin-5-one (XXII).

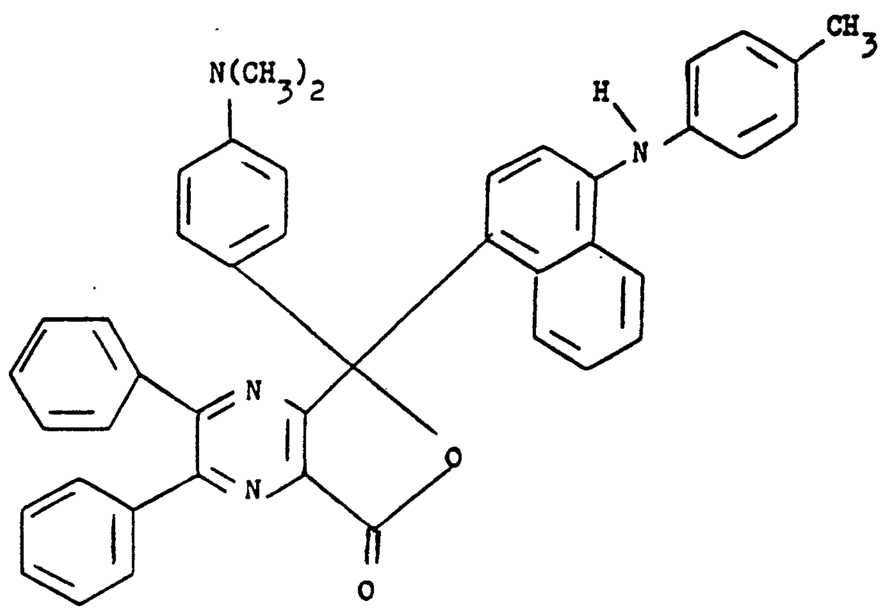

(XXII)

A mixture of (4-N,N-dimethylaminophenyl)-2-(carboxy-5,6-diphenylpyrazin-3-yl)ketone (1.0 g), (prepared by the method of Example 60), 4-methylanilinonaphthalene (0.6 g) and acetic anhydride (8 ml)

was stirred at 90°C for 3 hours. After cooling to room temperature, the solution was treated with 15% aqueous ammonia (50 ml), stirred for 2 hours. The product was filtered off and purified by column chromatography to yield compound (XXII).

A 1% solution of the product in a dialkyl naphthalene solvent showed little or no colouration on base paper.

$\lambda_{max}$ = 668 nm.

Example 166

Preparation of 2,3-dimethyl-7-(4-N,N-dimethylaminophenyl)-7-(1,2,2,4,7-pentamethyl-1,2-dihydroquinol-6-yl)-5,7-dihydrofuran[3,4-b]pyran-5-one (XXIII).

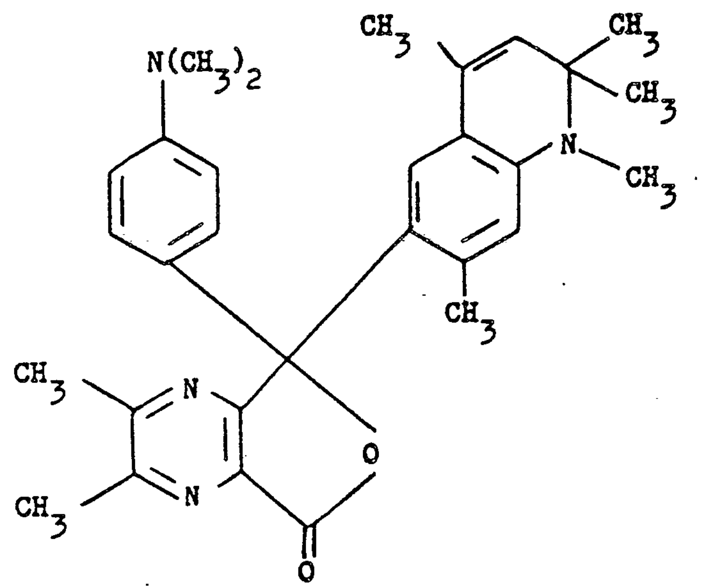

(XXIII)

A mixture of (4-N,N-dimethylaminophenyl)-(2-carboxy-5,6-dimethylpyrazin-3-yl)ketone (1.0 g) (prepared by the method of Example 61), 1,2,2,4,7-pentamethyl-1,2-dihydroquinoline (0.7 g) and acetic anhydride (8 ml) was stirred at 90°C for 2 hours. After cooling to room temperature, the solution was treated with 15% aqueous ammonia (100 ml) and stirred for 3 hours. The product was isolated by filtration and purified by column chromatography to yield compound (XXIII).

A 1% solution of the product in polyhalogenated paraffins showed little or no colouration on base paper.

$\lambda_{max}$ = 651 nm.

Example 167

Preparation of 2,3-dimethyl-7-(4-N,N-dimethylaminophenyl)-7-(1,2,2,4,7-pentamethyl-1,2,3,4-tetrahydroquinol-6-yl)-5,7-dihydrofuro[3,4-b]pyrazin-5-one (XXIV).

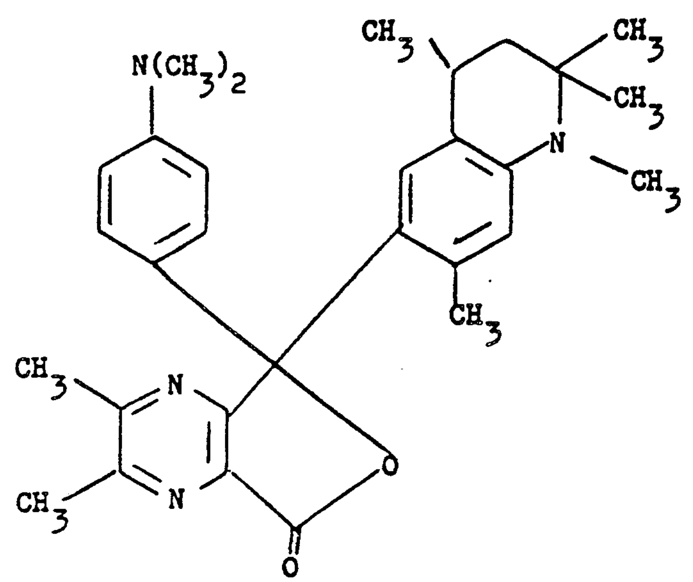

(XXIV)

A mixture of (4-N,N-dimethylaminophenyl)-(2--carboxy-5,6-dimethylpyrazin-3-yl)ketone (1.0 g)

(prepared by the method of Example 61), 1,2,2,4,7-pentamethyl-1,2,3,4-tetrahydroquinoline (0.7 g) and acetic anhydride (10 ml) was stirred at 90°C for 2 hours. After cooling to room temperature, the solution was treated with 15% aqueous ammonia (100 ml) and stirred for 5 hours. The product was isolated by filtration and purified by column chromatography to yield compound (XXIV).

A 1% solution of the product in polyhalogenated paraffins showed little or no colouration on base paper.

$\lambda_{max}$ = 649 nm.

Example 168

Preparation of 2,3-diphenyl-7-(4-N,N-dimethylaminophenyl)-7-(julolidin-9-yl)-5,7-dihydrofuro [3,4-b]pyrazin-5-one (XXV)>

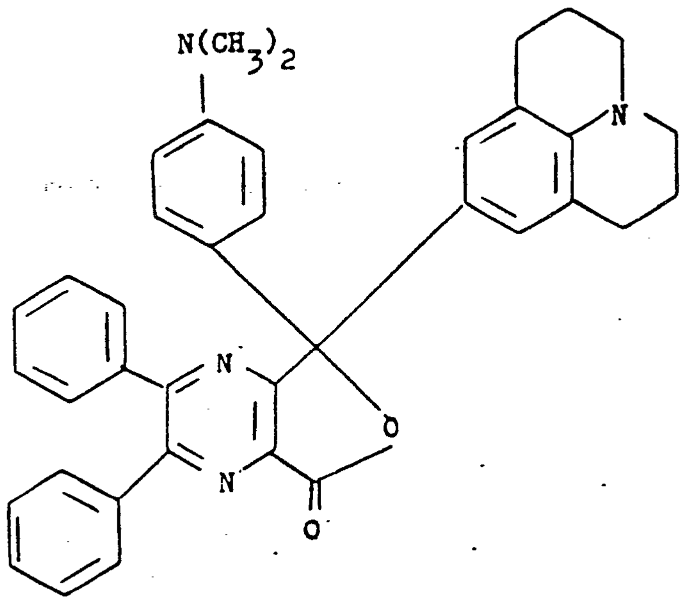

(XXV)

A mixture of (4-N,N-dimethylaminophenyl)-(2-carboxy-5,6-diphenylpyrazin-3-yl)ketone (0.5 g) (prepared by the method of Example 60), julolidine (0.4 g) and acetic anhydride (5 ml) was stirred at 85°C for 3 hours. After cooling to room temperature, the solution was treated with 15% aqueous ammonia (70 ml) and stirred for 3 hours. The product was filtered off and purified by column chromatography to yield compound (XXV).

A 1% solution of the product in monoisopropylbiphenyl showed little or no colour formation when applied to base paper.

$\lambda_{max}$ = 650 nm.

Example 169

Preparation of Heat-Sensitive Recording Paper

4 Parts of the colour former of Example 94 were milled with 10 parts of a 10% aqueous solution of polyvinyl alcohol and 6 parts of water to give Dispersion A. Separately, 4 parts of bisphenol A were milled with 10 parts of a 10% aqueous solution of polyvinyl alcohol and 6 parts of water to give Dispersion B.

1 Part of Dispersion A and 6 parts of Dispersion B were mixed, and the resulting mixture was coated onto a paper sheet support and dried to give heat-sensitive recording paper. When heat was applied by a heat pen to this heat-sensitive recording paper a green coloured image was produced. This coloured image has excellent light-fastness.

Example 170

Preparation of Pressure-Sensitive Copy Paper

The colour former of Example 23 was dissolved in an alkylnaphthalene solvent, and microencapsulated by a conventional method. The microcapsules were coated onto the surface of a paper sheet support, and dried to give an upper sheet (CB sheet). Correspondingly, a solid phenolic resin was coated onto the surface of a paper sheet support to give a lower sheet (CF sheet). When the CB sheet and the CF sheet were superposed so that both the coated surfaces faced each other, writing pressure on the upper surface of the CB sheet e.g. with a pen, gave a blue copied image on the lower sheet. This copied image is extremely lightfast.

**Claims**

1. A chromogenic pyrazine compound of formula:

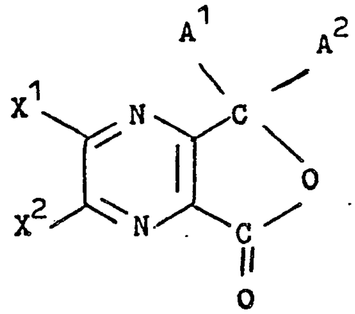

(I)

wherein $X^1$ and $X^2$, which may be the same or different, are each selected from hydrogen, alkyl, optionally substituted alkyl, alkoxy, carboxyalkyl, optionally substituted aryl, optionally substituted heteroaromatic, optionally substituted aralkyl or dialkylamino, provided that both $X^1$ and $X^2$ are not both hydrogen at the same time; and $A^1$ and $A^2$, which may be the same or different, are each selected from groups of the formulae:

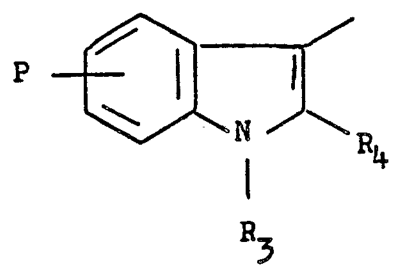

(II)

wherein $R_3$ and $R_4$ are each independently hydrogen, optionally substituted alkyl, optionally substituted aryl or optionally substituted aralkyl and P is hydrogen, alkyl, alkoxy, or halogen;

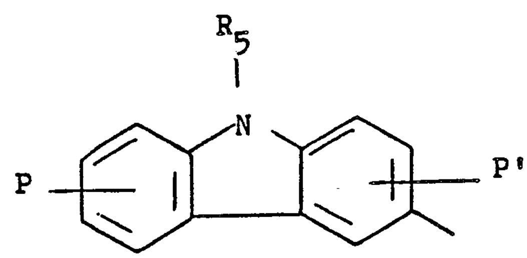

(III)

wherein P and P′, which may be the same or different, are optional substituents having the meaning given above for P; and $R_5$ is hydrogen, alkyl, aryl or aralkyl;

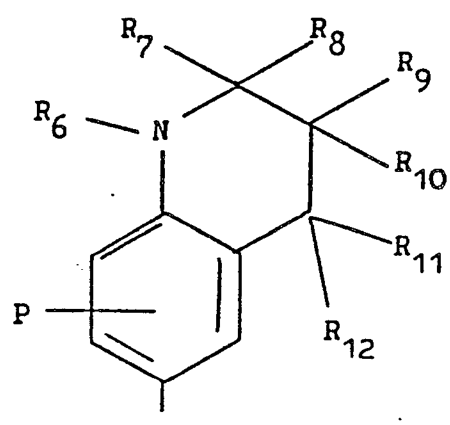

(IV)

wherein P is an optional substituent having the meaning given above, $R_6$ is hydrogen, alkyl, aryl or aralkyl, and the groups $R_7$ to $R_{12}$ are hydrogen, alkyl, aryl or aralkyl;

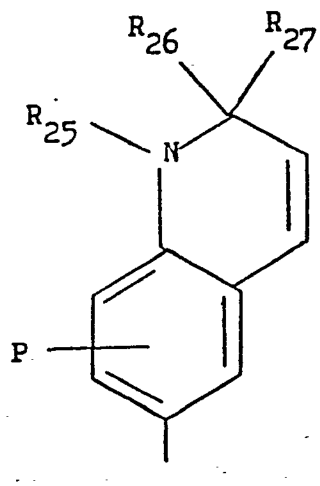

(IVa)

wherein P is an optional substituent having the meaning given above, $R_{25}$ is hydrogen or more preferably alkyl, aryl or aralkyl and the groups $R_{26}$ and $R_{27}$ are hydrogen, alkyl, aryl or aralkyl;

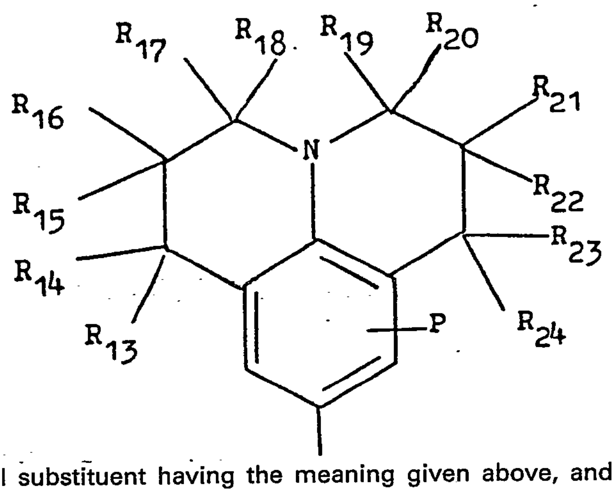

(V)

wherein P is an optional substituent having the meaning given above, and the groups $R_{13}$ to $R_{24}$ are hydrogen, alkyl, aryl or aralkyl;

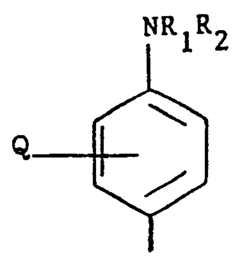

(VI)

wherein Q is hydrogen, alkyl, alkoxy, acylamino or halogen, and $R_1$ and $R_2$ are each independently hydrogen or an optionally substituted alkyl, aryl or aralkyl group or both $R_1$ and $R_2$ together with the nitrogen atom to which they are joined form a nitrogen-linked ring, provided that $R_1$ and $R_2$ not both hydrogen at the same time; and

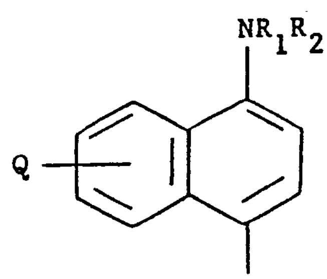

(VII)

wherein Q, and $R_1$ and $R_2$ have the meanings given above.

2. A chromogenic pyrazine compound according to claim 1 wherein each or $X_1$ and $X_2$ is optionally substituted $C_{1-8}$ alkyl or optionally substituted phenyl.

3. A chromogenic pyrazine compound according to claim 2 wherein $X^1$ and $X^2$ are identical.

4. A chromogenic pyrazine compound according to claim 3 wherein $X^1$ and $X^2$ are methyl or phenyl.

5. A chromogenic compound according to any preceding claim wherein each of $R_1$, $R_2$, $R_3$, $R_4$, $R_5$, $R_6$ and $R_{25}$ is $C_{1-6}$ alkyl, benzyl or phenyl.

6. A method for the preparation of a chromogenic pyrazine compound as defined in claim 1 which comprises reacting a compound of the formula:

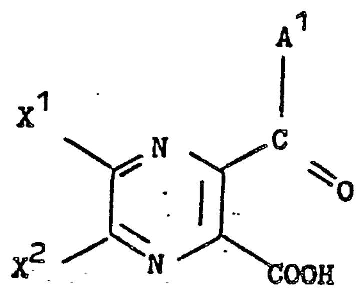

with a compound of the formula $A^2H$ in the presence of an acid condensing agent, the symbols $X^1$, $X^2$, $A^1$ and $A^2$ having the meanings given in claim 1.

7. Pressure-sensitive or heat-sensitive recording material which contains a chromogenic pyrazine compound as defined in claim 1.

**Patentansprüche**

1. Chromogene Pyrazin-Verbindung der Formel:

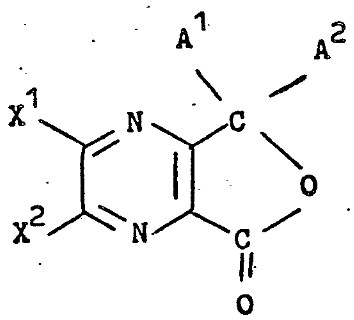

(I)

worin $X^1$ und $X^2$, welche gleich oder verschieden sein können, jeweils ausgewählt sind aus Wasserstoff, Alkyl, gegebenenfalls substituiertem Alkyl, Alkoxy, Carboxyalkyl, gegebenenfalls substituiertem Aryl, gegebenenfalls substituiertem Heteroaryl, gegebenenfalls substituiertem Aralkyl oder Dialkylamino, mit der Maßgabe, daß $X^1$ und $X^2$ nicht gleichzeitig für Wasserstoff stehen, und $A^1$ und $A^2$, welche gleich oder verschieden sein können, ausgewählt sind aus Gruppen der Formeln:

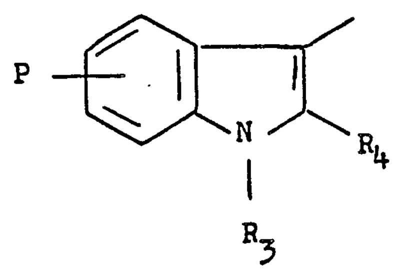

(II)

worin $R_3$ und $R_4$ unabhängig für Wasserstoff, gegebenenfalls substituiertes Alkyl, gegebenenfalls substituiertes Aryl oder gegebenenfalls substituiertes Aralkyl stehen und P für Wasserstoff, Alkyl, Alkoxy oder Halogen steht;

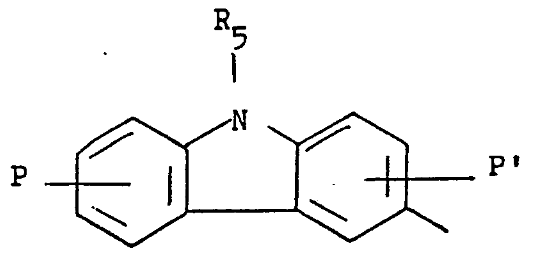

(III)

worin P und P', welche gleich oder verschieden sein können, für fakultative Substituenten stehen, welche die oben für P angegebenen Bedeutungen besitzen, und $R_5$ für Wasserstoff, Alkyl, Aryl oder Aralkyl steht;

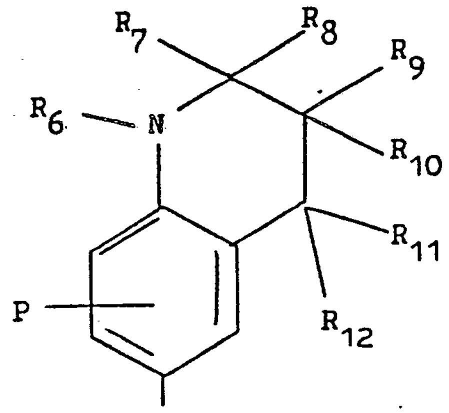

(IV)

worin P für einen fakultativen Substituenten steht, der die oben angegebene Bedeutung besitzt, $R_6$ für Wasserstoff, Alkyl, Aryl oder Aralkyl steht und die Gruppen $R_7$ bis $R_{12}$ für Wasserstoff, Alkyl, Aryl oder Aralkyl stehen;

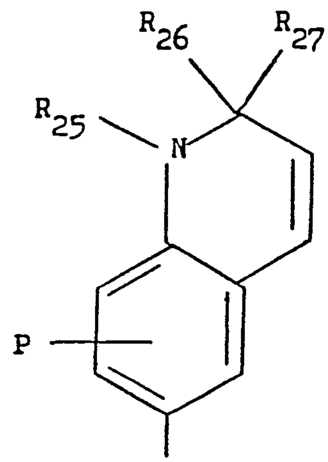

(IVa)

worin P für einen fakultativen Substituenten steht, der die oben angegebene Bedeutung besitzt, $R_{25}$ für Wasserstoff oder vorzugsweise Alkyl, Aryl oder Aralkyl steht und die Gruppen $R_{26}$ und $R_{27}$ für Wasserstoff, Alkyl, Aryl oder Aralkyl stehen;

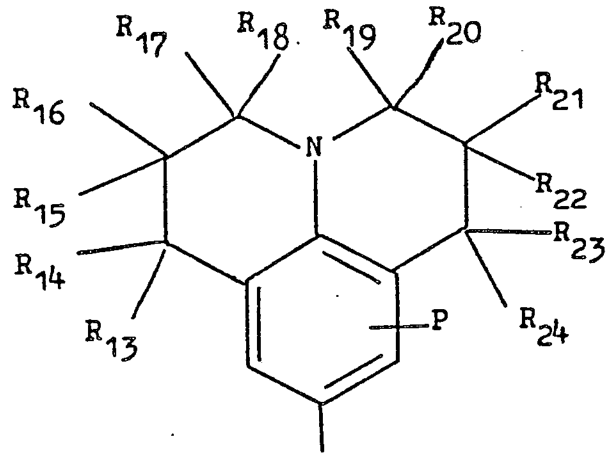

(V)

worin P für einen fakultativen Substituenten steht, der die oben angegebene Bedeutung besitzt, und die Gruppen $R_{13}$ bis $R_{24}$ für Wasserstoff, Alkyl, Aryl oder Aralkyl stehen;

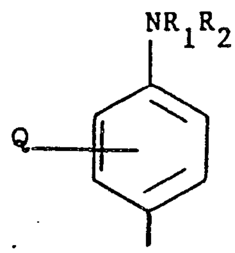

(VI)

worin Q für Wasserstoff, Alkyl, Alkoxy, Acylamino oder Halogen steht und $R_1$ und $R_2$ jeweils unabhängig für Wasserstoff oder eine gegebenenfalls substituierte Alkyl-, Aryl-, oder Aralkylgrupe stehen oder $R_1$ und $R_2$ gemeinsam mit dem Stickstoffatom, an welches sie gebunden sind, einen über Stickstoff gebundenen Ring bilden, mit der Maßgabe, daß $R_1$ und $R_2$ nicht gleichzeitig für Wasserstoff stehen; und

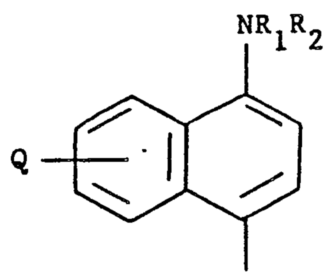

(VII)

worin Q, $R_1$ und $R_2$ die oben angegebenen Bedeutungen besitzen.

2. Chromogene Pyrazin-Verbindung nach Anspruch 1, bei welcher $X^1$ und $X^2$ jeweils für gegebenenfalls substituiertes $C_{1-8}$-Alkyl oder gegebenenfalls substituiertes Phenyl stehen.

3. Chromogene Pyrazin-Verbindung nach Anspruch 2, bei welcher $X^1$ und $X^2$ identisch sind.

4. Chromogene Pyrazin-Verbindung nach Anspruch 3, bei welcher $X^1$ und $X^2$ für Methyl oder Phenyl stehen.

5. Chromogene Pyrazin-Verbindung nach einem der vorhergehenden Ansprüche, bei welcher $R_1$, $R_2$, $R_3$, $R_4$, $R_5$, $R_6$ und $R_{25}$ jeweils für $C_{1-6}$-Alkyl, Benzyl oder Phenyl stehen.

6. Verfahren zur Herstellung einer chromogenen Pyrazin-Verbindung nach Anspruch 1, bei welchem eine Verbindung der Formel

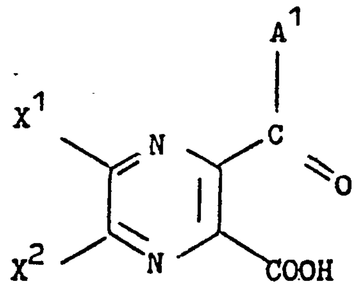

mit einer Verbindung der Formel $A^2H$ in Gegenwart eines sauren Kondensierungsmittels umgesetzt wird, wobei die Symbole $X^1$, $X^2$, $A^1$ und $A^2$ die in Anspruch 1 angegebenen Bedeutungen besitzen.

7. Druckempfindliches oder wärmeempfindliches Aufzeichnungsmaterial, welches eine chromogene Pyrazin-Verbindung nach Anspruch 1 enthält.

**Revendications**

1. Composé chromogène dérivé de la pyrazine, de formule:

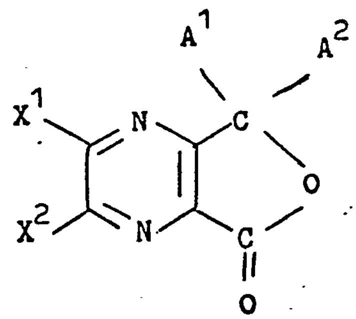

(I)

dans laquelle $X^1$ et $X^2$, qui peuvent être identiques ou différents, sont choisis chacun entre l'hydrogène, des groupes alkyle, alkyle facultativement substitué, alkoxy, carboxyalkyle, aryle facultativement substitué, hétéroaromatique facultativement substitué, aralkyle ou dialkylamino facultativement substitué, sous réserve que $X^1$ et $X^2$ ne représentent pas l'un et l'autre en même temps l'hydrogène; et $A^1$ et $A^2$, qui peuvent être identiques ou différents, sont choisis chacun entre:

des groupes de formules:

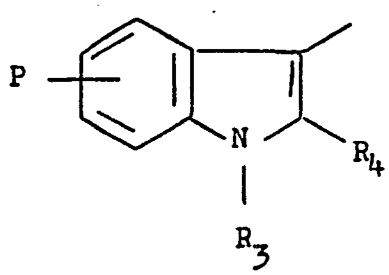

(II)

dans laquelle $R_3$ et $R_4$ représentent chacun indépendamment l'hydrogène, un groupe alkyle facultativement substitué, aryle facultativement substitué ou aralkyle facultativement substitué et P représente l'hydrogène, un groupe alkyle, alkoxy ou un halogène;

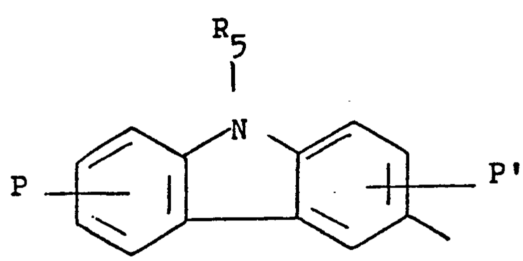

(III)

dans laquelle P et P', qui peuvent être identiques ou différents, représentent des substituants facultatifs répondant à la définition précitée pour P; et $R_5$ représente l'hydrogène, un groupe alkyle, alkyle ou aralkyle;

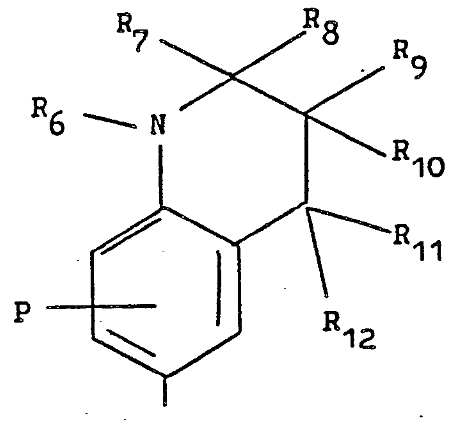

(IV)

dans laquelle P représente un substituant facultatif répondant à la définition précitée, $R_6$ représente l'hydrogène, un groupe alkyle, aryle ou aralkyle, et les groupes $R_7$ à $R_{12}$ représentent l'hydrogène, des groupes alkyle, aryle ou aralkyle;

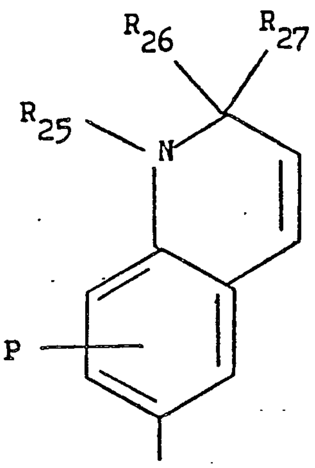

(IVa)

dans laquelle P représente un substituant facultatif répondant à la définition précitée, $R_{25}$ représente l'hydrogène ou, de préférence, un groupe alkyle, aryle ou aralkyle et les groupes $R_{26}$ et $R_{27}$ représentent l'hydrogène, des groupes alkyle, aryle ou aralkyle;

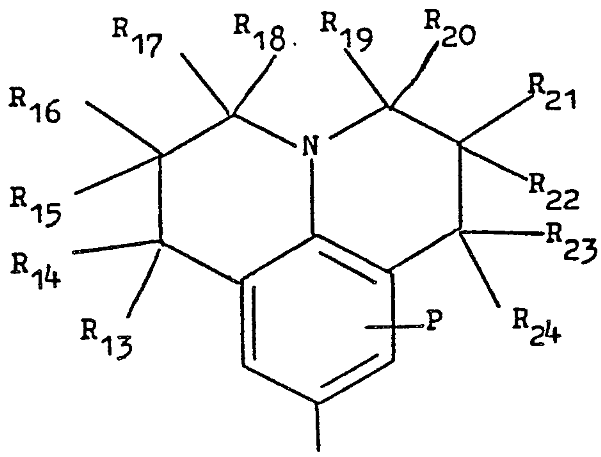

(V)

dans laquelle P représente un substituant facultatif répondant à la définition précitée et les groupes $R_{13}$ à $R_{24}$ représentent l'hydrogène, des groupes alkyle, aryle ou aralkyle;

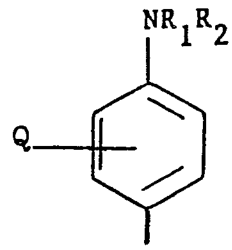

(VI)

dans laquelle Q représente l'hydrogène, un groupe alkyle, alkoxy, acylamino ou un halogène, et $R_1$ et $R_2$ représentent chacun indépendamment l'hydrogène ou un groupe alkyle, aryle ou aralkyle facultativement substitué, ou bien $R_1$ et $R_2$, conjointement avec l'atome d'azote auquel ils sont liés, forment un noyau dont la jonction est effectuée par l'atome d'azote, sous réserve que $R_1$ et $R_2$ ne représentent pas l'un et l'autre l'hydrogène en même temps; et

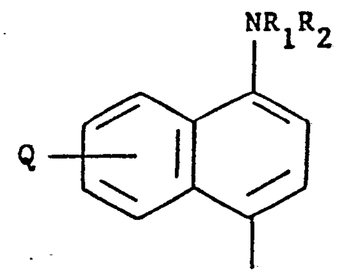

(VII)

dans laquelle Q et $R_1$ et $R_2$ répondent aux définitions précitées.

2. Composé chomogène dérivé de la pyrazine suivant la revendication 1, dans lequel chacun des substituants $X^1$ et $X^2$ représente un groupe alkyle en $C_1$ à $C_8$ facultativement substitué ou un groupe phényle facultativement substitué.

3. Composé chomogène dérivé de la pyrazine suivant la revendication 2, dans lequel $X^1$ et $X^2$ sont identiques.

4. Composé chomogène dérivé de la pyrazine suivant la revendication 3, dans lequel $X^1$ et $X^2$ représentent des groupes méthyle ou phényle.

5. Composé chromogène suivant l'une quelconque des revendications précédentes, dans lequel chacun des groupes $R_1$, $R_2$, $R_3$, $R_4$, $R_5$, $R_6$ et $R_{25}$ représente un groupe alkyle en $C_1$ à $C_6$, benzyle ou phényle.

6. Procédé de préparation d'un composé chromogène dérivé de la pyrazine suivant la revendication 1, qui consiste à faire réagir un composé de formule:

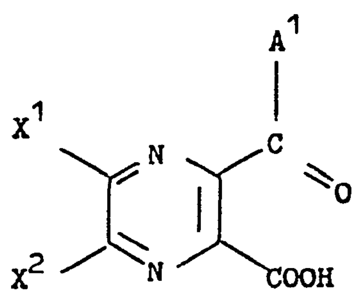

avec un composé de formule $A^2H$ en présence d'un agent de condensation acide, les symboles $X^1$, $X^2$, $A^1$ et $A^2$ répondant aux définitions mentionnées dans la revendication 1.

7. Matière d'enregistrement sensible à la pression ou thermosensible, qui contient un composé chromogène dérivé de la pyrazine suivant la revendication 1.